# EUROPEAN PATENT APPLICATION

(11) **EP 1 779 724 A1**
(43) Date of publication of application: **02.05.2007**
(21) Application number: 05767072.1
(22) Date of filing: 20.07.2005
(51) Int. Cl.: A01K 67/027, C12N 15/87, C12N 5/10, C12P 21/02

(54) **CONSTRUCTION OF CHIMERA USING ES CELLS**

(30) Priority: 20.07.2004 JP 2004211887
(71) Applicant: Nagao, Yasumitsu, Kyoto-shi, Kyoto 606-8237 (JP); Horii, Takuro, Maebashi-shi, Gunma 371-0031 (JP)
(72) Inventor: Nagao, Yasumitsu, Kyoto-shi, Kyoto 606-8237 (JP); Horii, Takuro, Maebashi-shi, Gunma 371-0031 (JP)
(74) Representative: Denison, Christopher Marcus
(86) International application number: PCT/JP2005/013685
(87) International publication number: WO 2006/009297

(57) **Abstract**

It is intended to provide a chimeric animal producing a protein or tissue of an animal of the same or a different species or comprising a desired pluripotent cell transmitted to the germ line and to provide a pluripotent cell having a deletion of a particular gene and lacking a function involving the gene. The present invention provides a method for producing a chimeric animal, comprising injecting into a host embryo of an animal, two or more kinds of cells comprising a pluripotent cell having a mutation or deletion of a particular gene and lacking a function involving the gene and an additional pluripotent cell other than the pluripotent cell, and a chimeric animal produced by the method.

## Description

### Technical Field

The present invention relates to a method for producing a chimeric animal by using a pluripotent cell having a mutation or deletion of a particular gene such as a gene involved in germ cell formation or a gene involved in the immune system.

### Background Art

To produce mice genetically modified by gene targeting such as knockout mice, chimeric mice have heretofore been formed with ES cells and mated.

The production of these genetically modified mice requires establishing excellent ES cells; efficiently performing a genetic modification in the ES cells, allowing the genetically modified ES cells to form chimeric mice, and transmitting the genetic modification performed in the ES cells to the germ lines of the obtained chimeric mice and as a result, to the next generations.

The establishment of ES cells depends largely on mouse strains. ES cells have a low chance of being established, or successfully established ES cells lose their abilities to be transmitted to germ lines as subculture is repeated. Therefore, even ES cells established by expending enormous efforts had to be used with minimal subculture. This tendency was particularly significant in using inbred mice.

The transmission of a genetic modification of ES cells to germ lines presented many problems. Particularly, chimeric mice, even if successfully produced, often showed no transmission to germ lines. In fact, ES cells with minimal subculture proved to be transmitted to germ lines were obtained and used.

A tetraploid rescue method has been developed as a method for selectively producing chimeric mice from ES cells (Nagy A et al., Development 110, 815-821 (1990)). In the method, ES cells are injected into tetraploid fertilized eggs, and a genetic modification in the ES cells can be transmitted to germ lines because tetraploid cells develop into placenta while only ES cells develop into individuals.

However, this method produces chimeric mice that cannot live long due to the onset of respiratory disorder or disorder such as malformation (Eggan K et al., PNAS 98, 6209-6214 (2001)) and could not serve as an effective method for producing germ line chimeras.

The present inventor has solved these problems and developed methods for effectively producing a germ line chimera. One of them was a method for producing a chimeric mouse having only an ES cell-derived germ cell by injecting an ES cell into a mouse early embryo incapable of forming a germ cell, wherein the mouse early embryo incapable of forming a germ cell is a fertilized embryo (W^{v}/W^{v} or W/W^{v}) obtained from the reciprocal cross of c-kit mutant mice, a W^{v}/+ mouse and a W/+ mouse, and is a mouse hermaphroditic early embryo incapable of forming a germ cell (sterile/infertile) (see Patent Document 1).

Moreover, the present inventors have developed a method for producing a chimeric mouse without the onset of respiratory disorder or disorder such as malformation even in the tetraploid rescue method by substituting wild-type mitochondrial DNA for the mitochondrial DNA of an ES cell or a cell of an early embryo into which the ES cell is injected and thereby adapting the mitochondrial DNA to nuclear DNA (see Patent Document 2).

However, even these methods were not effective in light of ES cell preparation because the ES cell used had to maintain its proliferating ability, undifferentiated form, and differentiation potency without repeating subculture. Moreover, it was impossible to control the sex ratio of born chimeric mice.
Patent Document 1: Pamphlet of International Publication of WO 03/071869
Patent Document 2: Pamphlet of International Publication of WO 03/072777

### Disclosure of the Invention

An object of the present invention is to provide a novel method for producing a chimeric animal by using two or more kinds of cells comprising a pluripotent cell having a mutation or deletion of a particular gene and lacking a function involving the gene and an additional pluripotent cell having the function and to provide a chimeric animal produced by the method, which produces a protein or tissue of an animal of the same or a different species or comprises a desired pluripotent cell transmitted to the germ line. A further object of the present invention is to provide a pluripotent cell having a deletion of a particular gene and lacking a function involving the gene and to provide a method for establishing an additional pluripotent cell by using the pluripotent cell.

As described above, a method has been established whereby a germ line chimeric mouse is efficiently produced by modifying the mitochondrion of an ES cell or using an embryo derived from a mouse incapable of forming a germ cell such as a W^{v}/W mouse (W/W^{v} mouse) as well as an ES cell.

However, the preparation of a fertilized embryo derived from a mouse incapable of forming a germ cell such as a W^{v}/W mouse (W/W^{v} mouse) required enormous efforts and was therefore difficult.

ES cells used for producing chimeric mice are desired to have a high proliferating ability. However, the reality is that ES cells having a high proliferating ability were not easily established, and even established ES cells presented the problem of a reduction in their proliferating abilities as subculture is repeated, with the result that chimeric mice could not be produced efficiently. ES cells conventionally obtained have male XY sex chromosomes. Therefore, most of chimeric mice are male when the ES cells significantly contribute thereto. Thus, the conventional methods could not control the sex ratio of born chimeric mice.

It has been known that ES cells of inbred mice show a reduction in their proliferating abilities as subculture is repeated, and however, this reduction in their proliferating abilities is prevented by coculturing the cells of F1 hybrid mice therewith (Axelrod. Dev Biol 101, 225-228, 1984).

The present inventors have conducted diligent studies and have consequently found out that an ES cell is efficiently established from a reproductive organ-derived cell by establishing an ES cell from a mouse embryo incapable of forming a germ cell and coculturing the ES cell with a cell derived from the reproductive organ of an animal. In this manner, the above-described difficulty in preparing a fertilized embryo derived from a mouse incapable of forming a germ cell can be solved by establishing an ES cell from a mouse embryo incapable of forming a germ cell and simultaneously injecting the ES cell and a desired ES cell into a host embryo. The present inventors have further found out that the proliferating ability of an additional ES cell can be improved by coculturing the ES cell established from the mouse incapable of forming a germ cell with the additional ES cell, for example, a genetically modified ES cell. The present inventors have further found out that a chimeric animal comprising an additional ES cell transmitted to the germ line can be produced by injecting the ES cell established from the mouse embryo incapable of forming a germ cell together with the additional ES cell, for example, a genetically modified ES cell, into an early embryo of an animal, and then causing ontogenesis. In this manner, an ES cell capable of forming a germ cell is transmitted to the germ line by injecting into an early embryo of an animal, a mouse embryo-derived ES cell having a mutation in a gene involved in germ cell formation and being incapable of forming a germ cell and the ES cell capable of forming a germ cell, and then causing ontogenesis. Therefore, progeny of the chimeric individual is derived from the ES cell capable of forming a germ cell.

Furthermore, the present inventors have completed the present invention by finding out that an additional pluripotent cell other than a pluripotent cell having a mutation or deletion of a particular gene and lacking a function involving the gene can function in a chimeric individual by injecting into an early embryo of an animal, the animal-derived pluripotent cell having a mutation or deletion of a particular gene and having an impairment of a function involving the gene, such as an ES cell derived from an embryo of an immunodeficient mouse, lymphocyte-deficient mouse, or the like, and the additional pluripotent cell, and then causing ontogenesis.

Specifically, the present invention is as follows:
[1] A method for producing a chimeric animal, comprising injecting into a host embryo of an animal, two or more kinds of cells comprising a pluripotent cell having a mutation or deletion of a particular gene and lacking a function involving the gene and an additional pluripotent cell other than the pluripotent cell.
[2] A method for producing a chimeric animal, comprising coculturing two or more kinds of cells comprising a pluripotent cell having a mutation or deletion of a particular gene and lacking a function involving the gene and an additional pluripotent cell other than the pluripotent cell, and then simultaneously injecting the cells into a host embryo of an animal.
[3] The method for producing a chimeric animal according to [2], wherein the coculture is performed in an early embryo of an animal.
[4] The method for producing a chimeric animal according to any of [1] to [3], wherein the chimeric animal is a chimeric animal where a gene functions which is derived from the additional cell other than the pluripotent cell having a mutation or deletion of a particular gene and lacking a function involving the gene.
[5] The method for producing a chimeric animal according to any of [1] to [3], wherein the chimeric animal is a chimeric animal where a gene functions which is derived from the additional cell other than the pluripotent cell having a mutation or deletion of a particular gene and lacking a function involving the gene and corresponds to the gene mutated or deleted.
[6] The method for producing a chimeric animal according to any of [1] to [5], wherein the additional pluripotent cell other than the pluripotent cell having a mutation or deletion of a particular gene and lacking a function involving the gene is a genetically modified cell.
[7] The method for producing a chimeric animal according to [6], wherein the genetic modification is introduction of a foreign gene or knockout of an endogenous gene.
[8] The method for producing a chimeric animal according to any of [1] to [7], wherein the pluripotent cell having a mutation or deletion of a particular gene and lacking a function involving the gene and/or the additional pluripotent cell are pluripotent cells comprising adapted mitochondrial DNA introduced therein or substituted for the original mitochondrial DNA.
[9] The method for producing a chimeric animal according to any of [1] to [8], wherein the host embryo of the animal is a host embryo comprising adapted mitochondrial DNA introduced therein.
[10] The method for producing a chimeric animal according to any of [1] to [9], wherein the host embryo of the animal is a blastocyst.
[11] The method for producing a chimeric animal according to [10], wherein the host embryo of the animal is a tetraploid.
[12] The method for producing a chimeric animal according to any of [1] to [11], wherein the pluripotent cell is a cell selected from the group consisting of an ES cell, an EG cell, and a GS cell.
[13] The method for producing a chimeric animal according to any of [1] to [12], wherein the additional cell other than the pluripotent cell having a mutation or deletion of a particular gene and lacking a function involving the gene is of the same species in terms of zootaxy as the pluripotent cell having a mutation or deletion of a particular gene and lacking a function involving the gene.
[14] The method for producing a chimeric animal according to any of [1] to [12], wherein the additional cell other than the pluripotent cell having a mutation or deletion of a particular gene and lacking a function involving the gene is of a different species in terms of zootaxy from the pluripotent cell having a mutation or deletion of a particular gene and lacking a function involving the gene.
[15] The method for producing a chimeric animal according to any of [1] to [14], wherein the pluripotent cell having a mutation or deletion of a particular gene and lacking a function involving the gene is derived from an animal selected from the group consisting of a mouse, a rat, a pig, cattle, a horse, a sheep, a goat, and a monkey.
[16] The method for producing a chimeric animal according to any of [1] to [15], wherein the additional cell other than the pluripotent cell having a mutation or deletion of a particular gene and lacking a function involving the gene is derived from an animal selected from the group consisting of a mouse, a rat, a pig, cattle, a horse, a sheep, a goat, a monkey, and a human.
[17] The method for producing a chimeric animal according to any of [1] to [16], wherein in the pluripotent cell having a mutation or deletion of a particular gene and lacking a function involving the gene, the gene mutated or deleted is one or more genes selected from the group consisting of
   (a) a gene involved in germ cell formation,
   (b) a gene involved in the immune system, and
   (c) a gene involved in formation of a particular tissue or organ or a gene encoding a particular protein.
[18] The method for producing a chimeric animal according to any of [1] to [16], wherein the pluripotent cell having a mutation or deletion of a particular gene and lacking a function involving the gene is a pluripotent cell incapable of forming a germ cell, which is obtained from interspecific crossing.
[19] The method for producing a chimeric animal according to [17], wherein the pluripotent cell having a mutation or deletion of a particular gene and lacking a function involving the gene is a pluripotent cell having a mutation or deletion of a gene involved in germ cell formation and being incapable of forming a germ cell, and the additional pluripotent cell other than the pluripotent cell having a mutation or deletion of a particular gene and lacking a function involving the gene is transmitted to the germ line.
[20] The method for producing a chimeric animal according to [19], wherein the pluripotent cell having a mutation or deletion of a gene involved in germ cell formation and being incapable of forming a germ cell is a C-kit mutant animal-derived pluripotent cell incapable of forming a germ cell.
[21] The method for producing a chimeric animal according to [20], wherein the C-kit mutant animal is a W^{v}/W^{v} mouse.
[22] The method for producing a chimeric animal according to any of [19] to [21], wherein the additional pluripotent cell other than the pluripotent cell having a mutation or deletion of a particular gene and lacking a function involving the gene is a genetically modified cell, and the genetically modified pluripotent cell is transmitted to the germ line.
[23] The method for producing a chimeric animal according to [22], wherein the genetic modification is introduction of a foreign gene or knockout of an endogenous gene.
[24] The method for producing a chimeric animal according to [17], wherein the pluripotent cell having a mutation or deletion of a particular gene and lacking a function involving the gene is a pluripotent cell having a mutation or deletion of a gene involved in the immune system and having an impairment of immune function.
[25] The method for producing a chimeric animal according to [24], wherein the pluripotent cell having a mutation or deletion of a gene involved in the immune system and having an impairment of immune function is a pluripotent cell having a mutation or deletion of a gene encoding an antibody and lacking an ability to produce an antibody.
[26] The method for producing a chimeric animal according to [24], wherein the pluripotent cell having a mutation or deletion of a gene involved in the immune system and having an impairment of immune function is an immunodeficient animal-derived pluripotent cell.
[27] The method for producing a chimeric animal according to [26], wherein the immunodeficient animal is a nu/nu mouse or scid mouse.
[28] The method for producing a chimeric animal according to [24], wherein the pluripotent cell having a mutation or deletion of a gene involved in the immune system and having an impairment of immune function is a lymphocyte-deficient animal-derived pluripotent cell.
[29] The method for producing a chimeric animal according to [28], wherein the lymphocyte-deficient animal is an animal having a mutation or deletion of a RAG-1 gene and/or a RAG-2 gene.
[30] The method for producing a chimeric animal according to [17], wherein the pluripotent cell having a mutation or deletion of a particular gene and lacking a function involving the gene is a pluripotent cell having a mutation or deletion of a gene involved in formation of a particular tissue or organ or a gene encoding a particular protein.
[31] The method for producing a chimeric animal according to [30], wherein a gene which is carried by the additional pluripotent cell other than the pluripotent cell having a mutation or deletion of a particular gene and lacking a function involving the gene and corresponds to the gene mutated or deleted causes a particular tissue or organ or particular protein involving the gene to be produced in the produced chimeric animal.
[32] The method for producing a chimeric animal according to [17], wherein the pluripotent cell having a mutation or deletion of a particular gene and lacking a function involving the gene is a pluripotent cell having a mutation or deletion of a gene involved in the immune system and further having a mutation or deletion of a gene involved in germ cell formation or a gene involved in formation of a particular tissue or organ or a gene encoding a particular protein.
[33] The method for producing a chimeric animal according to any of [1] to [32], wherein the additional pluripotent cell other than the pluripotent cell having a mutation or deletion of a particular gene and lacking a function involving the gene is
   (a) a fusion cell between the additional pluripotent cell other than the pluripotent cell having a mutation or deletion of a particular gene and lacking a function involving the gene and a cell of the same or a different species in term of zootaxy as (from) the pluripotent cell having a mutation or deletion of a particular gene, or
   (b) a pluripotent cell derived from a cell of the same or a different species in terms of zootaxy as (from) the pluripotent cell having a mutation or deletion of a particular gene.
[34] The method for producing a chimeric animal according to [33], wherein the cell of the same or a different species in terms of zootaxy as (from) the additional pluripotent cell other than the pluripotent cell having a mutation or deletion of a particular gene and lacking a function involving the gene is a tissue stem cell or differentiated cell.
[35] The method for producing a chimeric animal according to [33] or [34], wherein the method comprises injecting into the host embryo of the animal, a pluripotent cell having a mutation or deletion of a gene involved in germ cell formation and being incapable of forming a germ cell, and
   (a) a fusion cell between the pluripotent cell having a mutation or deletion of a gene involved in germ cell formation and being incapable of forming a germ cell and a cell of the same or a different species in terms of zootaxy as (from) the pluripotent cell having a mutation or deletion of a gene involved in germ cell formation, or
   (b) a pluripotent cell-like cell derived from a cell of the same or a different species in terms of zootaxy as (from) the pluripotent cell having a mutation or deletion of a gene involved in germ cell formation,
   wherein the cell of the same or a different species is capable of being transmitted to the germ line.
[36] The method for producing a chimeric animal according to [33] or [34], wherein the method comprises injecting into the host embryo of the animal, a pluripotent cell having a mutation or deletion of a gene involved in the immune system and having an impairment of immune function, and
   (a) a fusion cell between the pluripotent cell having a mutation or deletion of a gene involved in the immune system and having an impairment of immune function and a cell of a different species in terms of zootaxy from the pluripotent cell, or
   (b) a pluripotent cell-like cell derived from a cell of a different species from the pluripotent cell,
   wherein the pluripotent cell having a mutation or deletion of a gene involved in the immune system and having an impairment of immune function and the cell of a different species contribute to formation of the chimeric individual.
[37] A chimeric animal produced by a method according to any of [1] to [36].
[38] A method for producing a germ cell derived from an additional pluripotent cell other than a pluripotent cell having a mutation or deletion of a particular gene and lacking a function involving the gene, comprising collecting from a chimeric animal produced by a method according to any of [17] to [23], a germ cell derived from an additional pluripotent cell other than a pluripotent cell having a mutation or deletion of a particular gene and lacking a function involving the gene.
[39] A method for producing a genetically modified animal, comprising mating a chimeric animal produced by a method according to any of [17] to [23] and obtaining a genetically modified animal as progeny.
[40] A genetically modified animal produced by a method according to [39].
[41] A method for producing an antibody, comprising administering an antigen to a chimeric animal or progeny thereof produced by a method according to any of [17] and [24] to [29],
   wherein the chimeric animal or progeny thereof has a gene encoding an antibody derived from an additional pluripotent cell other than a pluripotent cell having a mutation or deletion of a gene involved in the immune system and having an impairment of immune function.
[42] A method for producing a particular tissue or organ or a particular protein, comprising allowing a chimeric animal or progeny thereof produced by a production method according to any of [17], [30], and [31] to form a particular tissue or organ or to produce a particular protein.
[43] A pluripotent cell derived from a C-kit mutant animal which is incapable of forming a germ cell.
[44] The pluripotent cell according to [43], wherein the C-kit mutant animal is a W^{v}/W^{v} mouse.
[45] A pluripotent cell derived from an immunodeficient animalwhich has a mutation or deletion of a gene involved in immunity.
[46] The pluripotent cell according to [45], wherein the animal is a nu/nu mouse or scid mouse.
[47] A pluripotent cell derived from a lymphocyte-deficient animalwhich has a mutation or deletion of a gene involved in immunity.
[48] The pluripotent cell according to [47], wherein the lymphocyte-deficient animal is an animal having a mutation or deletion of a RAG-1 gene and/or a RAG-2 gene.
[49] The pluripotent cell according to any of [43] to [48], wherein the pluripotent cell is a cell selected from the group consisting of an ES cell, an EG cell, and a GS cell.
[50] The pluripotent cell according to any of [43] to [49], wherein the pluripotent cell comprises adapted mitochondrial DNA introduced therein as mitochondrial DNA.
[51] The pluripotent cell according to [50], wherein the adapted mitochondrial DNA is a wild-type mouse Mus musculus musculus type.
[52] The pluripotent cell according to any of [43] to [51], wherein the sex chromosomal type is a male XY type.
[53] The pluripotent cell according to any of [43] to [51], wherein the sex chromosomal type is a female XX type.
[54] A method for establishing a pluripotent cell from a cell derived from an early embryo or germ cell by mixed-culturing a pluripotent cell according to any of [43] to [53] with an animal reproductive organ-derived cell.
[55] The method according to [54], wherein the early embryo of the animal is an androgenetic embryo or gynogenetic embryo.
[56] The method for establishing a pluripotent cell from a cell derived from an early embryo or germ cell according to [54] or [55], wherein the mixed-culture is coculture in an early embryo of an animal.
[57] The method for establishing a pluripotent cell according to any of [54] to [56], wherein the cell derived from an early embryo or germ cell is a cell derived from an inner cell mass of a blastocyst, and the pluripotent cell is an ES cell.
[58] The method for establishing a pluripotent cell according to any of [54] to [56], wherein the cell derived from an early embryo or germ cell is a primordial germ cell, and the pluripotent cell is an EG cell.
[59] The method for establishing a pluripotent cell according to any of [54] to [56], wherein the cell derived from an early embryo or germ cell is a germ cell, and the pluripotent cell is a GS cell.
[60] A method for improving a proliferating ability of an additional pluripotent cell, comprising coculturing a pluripotent cell according to any of [43] to [53] with the additional pluripotent cell.
[61] The method for improving a proliferating ability of an additional pluripotent cell according to [60], wherein the coculture is performed in an early embryo of an animal.
[62] The method for improving a proliferating ability of a pluripotent cell according to [60] or [61], wherein the additional pluripotent cell is a cell selected from the group consisting of an ES cell, an EG cell, and a GS cell.
[63] The method for improving a proliferating ability of a pluripotent cell according to any of [60] to [62], wherein the additional pluripotent cell is a genetically modified pluripotent cell.
[64] A method for producing a germ line chimeric animal comprising a germ cell derived from an additional pluripotent cell, comprising injecting a pluripotent cell according to any of [43] to [53] together with the additional pluripotent cell into a host embryo of an animal.
[65] The method for producing a germ line chimeric animal according to [64], wherein the pluripotent cell according to any of [43] to [53] and/or the additional pluripotent cell are pluripotent cells comprising adapted mitochondrial DNA introduced therein or substituted for the original mitochondrial DNA.
[66] The method for producing a germ line chimeric animal according to [64] or [65], wherein the host embryo of the animal is a host embryo comprising adapted mitochondrial DNA introduced therein or substituted for the original mitochondrial DNA.
[67] The method for producing a germ line chimeric animal according to any of [64] to [66], wherein the host embryo of the animal is a blastocyst.
[68] The method for producing a germ line chimeric animal according to [67], wherein the host embryo of the animal is a tetraploid.
[69] The method for producing a germ line chimeric animal according to any of [64] to [68], wherein the additional pluripotent cell is a cell selected from the group consisting of an ES cell, an EG cell, and a GS cell.
[70] The method for producing a germ line chimeric animal according to any of [64] to [69], wherein the animal is a mouse.
[71] The method for producing a germ line chimeric animal according to any of [64] to [70], wherein the additional pluripotent cell is a genetically modified pluripotent cell.
[72] A method for producing a germ line chimeric animal comprising a germ cell derived from an additional pluripotent cell, comprising coculturing a pluripotent cell according to any of [43] to [53] with the additional pluripotent cell and injecting the cocultured cells together into a host embryo of an animal.
[73] The method for producing a germ line chimeric animal according to [72], wherein the coculture is performed in an early embryo of an animal.
[74] The method for producing a germ line chimeric animal according to [72] or [73], wherein the pluripotent cell according to any of [43] to [53] and the additional pluripotent cell are pluripotent cells comprising adapted mitochondrial DNA introduced therein.
[75] The method for producing a germ line chimeric animal according to any of [72] to [74], wherein the host embryo of the animal is a host embryo comprising adapted mitochondrial DNA introduced therein.
[76] The method for producing a germ line chimeric animal according to any of [72] to [75], wherein the host embryo of the animal is a blastocyst.
[77] The method for producing a germ line chimeric animal according to [76], wherein the host embryo of the animal is a tetraploid.
[78] The method for producing a germ line chimeric animal according to any of [72] to [77], wherein the additional pluripotent cell is a cell selected from the group consisting of an ES cell, an EG cell, and a GS cell.
[79] The method for producing a germ line chimeric animal according to any of [72] to [78], wherein the animal is a mouse.
[80] The method for producing a germ line chimeric animal according to any of [72] to [79], wherein the additional pluripotent cell is a genetically modified pluripotent cell.
[81] A chimeric animal produced by a method according to any of [64] to [80].
[82] A method for producing a germ cell by transplanting an androgenetic embryo- or gynogenetic embryo-derived pluripotent cell established by a method according to [55] into the reproductive organ of an animal.
[83] The method according to [82], wherein a sperm is produced by transplanting the androgenetic embryo-derived ES cell to the vas deferens of the animal.

The present specification encompasses the contents described in the specification and/or drawings of Japanese Patent Application No. 2004-211887 that serves as a basis of the priority of the present application.

### Brief Description of the Drawings

Figure 1 is a diagram showing the number of transplantations, the number of newborns, and so on in the production of chimeric mice by a method of the present invention using 129.B6-YGFP ES cells and W^{v}/W^{v} ES cells;
Figure 2 is photographs of chimeric mice produced by the method of the present invention by using 129.B6-YGFP ES cells and W^{v}/W^{v} ES cells;
Figure 3 is photographs showing the fluorescence of the chimeric mice produced by the method of the present invention by using 129.B6-YGFP ES cells and W^{v}/W^{v} ES cells;
Figure 4 is photographs of chimeric mice produced by the method of the present invention by using BS-neo ES cells and W^{v}/W^{v} ES cells;
Figure 5 is a diagram showing the outline of chimera production by the method of the present invention (Example 7);
Figure 6 is a diagram showing the outline of chimera production by the method of the present invention (Example 8);
Figure 7 is a diagram showing the outline of the method of the present invention; and
Figure 8 is a photograph showing spermatogenesis from androgenetic embryo-derived ES cells.

### Best Mode for Carrying Out the Invention

In a method of the present invention for producing a chimeric animal by using two or more kinds of cells comprising a pluripotent cell having a mutation or deletion of a particular gene and lacking a function involving the gene and an additional pluripotent cell other than the pluripotent cell, the gene mutated or deleted is not limited and encompasses any gene as long as the gene loses a particular function due to the mutation or deletion of the gene. In this context, the phrase "having a mutation or deletion of a particular gene and lacking a function involving the gene" means that a deletion, substitution, or the like occurs in the partial or whole nucleotide sequence of the gene or the addition or the like of a base occurs in the gene, resulting in a loss or reduction of the function of the gene. It encompasses both the case where the pluripotent cell itself loses the function and the case where a chimeric individual or progeny individual thereof derived from the pluripotent cell loses the function. The "function of the gene" encompasses all functions involving the gene, such as the expression of the gene and the following in-vivo production of a particular substance or the following *in-vivo* formation of a particular organ, tissue, or the like, and as the expression of the gene and the following in-vivo production of a particular substance, followed by a particular *in-vivo* reaction caused by the substance. The "pluripotent cell having a mutation or deletion of a particular gene and lacking a function involving the gene" of the present invention is a "pluripotent cell having a deletion of a gene involved in a cell, tissue, organ, protein, or the like, of interest". In this context, the cell, tissue, organ, or protein of interest refers to a visceral organ or protein desired to be produced by a chimeric animal.

The pluripotent cell of the present invention refers to a cell capable of differentiating into all cells. The pluripotent cell includes a totipotent cell capable of differentiating into a germ cell and a tissue stem cell capable of differentiating into an animal tissue. The totipotent cell includes an embryonic stem cell (ES cell), an EG cell (embryonic germ cell), and a GS cell. In this context, an inner cell mass-derived cell is referred to as an ES cell; a primordial germ cell-derived cell is referred to as an EG cell; and a germ cell-derived cell is referred to as a GS cell. Alternatively, a cell capable of differentiating into a germ cell, which is similar in property to the totipotent cell and has pluripotency, is also referred to as a totipotent cell-like cell, for example, an ES cell-like cell, (ES-like cell), an EG cell like-cell (EG-like cell), and a GS cell-like cell (GS-like cell). The pluripotent cell used in the present invention also encompasses these cells called pluripotent cell-like cells. For example, the ES cell-like cell is obtained by transforming a tissue stem cell or differentiated somatic cell of an animal or fusing an ES cell with a tissue stem cell or differentiated somatic cell of an animal.

A chimeric individual obtained by the method of the present invention is formed at least from a cell such as a differentiated cell derived from the pluripotent cell having a mutation or deletion of a particular gene and lacking a function involving the gene and from a cell such as a differentiated cell derived from the additional pluripotent cell. When a cell other than these two kinds of cells is used in the chimeric animal production, the chimeric individual is composed of the two kinds of cells and the cell other than the two kinds of cells. The "cell other than the two kinds of cells" is not limited, and examples thereof include a tissue stem cell of an animal and a differentiated somatic cell of an animal.

In the chimeric individual, a cell other than a cell derived from the pluripotent cell having a mutation or deletion of a particular gene and lacking a function involving the gene, a chromosome retained by the cell, and a gene retained by the cell are functioning. The additional pluripotent cell other than the pluripotent cell having a mutation or deletion of a particular gene and lacking a function involving the gene includes both a pluripotent cell of a cell derived from an animal of a different species in terms of zootaxy from the pluripotent cell having a mutation or deletion of a particular gene and lacking a function involving the gene and a pluripotent cell derived from an animal of the same species in terms of zootaxy as the pluripotent cell. In this context, the phrase "a cell other than a cell derived from the pluripotent cell having a mutation or deletion of a particular gene and lacking a function involving the gene, a chromosome retained by the cell, and a gene retained by the cell are functioning" means that, for example, the cell other than a cell derived from the pluripotent cell produces a particular substance or forms a particular organ or tissue. The "gene" in the phrase "and a gene retained by the cell are functioning" may be a gene identical to or different from the particular gene mutated or deleted in the "pluripotent cell having a mutation or deletion of a particular gene and lacking a function involving the gene". Preferably, a gene is functioning which is retained by the cell other than a cell derived from the pluripotent cell having a mutation or deletion of a particular gene and lacking a function involving the gene and corresponds to the gene mutated or deleted. In this context, the "gene which corresponds to the gene mutated or deleted" refers to a gene identical to or highly homologous in nucleotide sequence to the gene mutated or deleted, wherein the gene has a function identical or similar to the function involving the gene mutated or deleted.

Examples of the pluripotent cell having a mutation or deletion of a particular gene and lacking a function involving the gene include a pluripotent cell having a mutation or deletion of a gene involved in germ cell formation, a pluripotent cell having a mutation or deletion of a gene involved in the immune system, and a pluripotent cell having a mutation or deletion of other genes such as a gene involved in formation of a particular tissue or organ or a gene encoding a particular protein. Specifically, in a pluripotent cell that can be used as the pluripotent cell having a mutation or deletion of a particular gene and lacking a function involving the gene, the gene mutated or deleted is one or more genes selected from the group consisting of (a) a gene involved in germ cell formation, (b) a gene involved in the immune system, and (c) a gene involved in formation of a particular tissue or organ or a gene encoding a particular protein.

In this case, the additional pluripotent cell other than the pluripotent cell having a mutation or deletion of a particular gene and lacking a function involving the gene is a pluripotent cell free from the mutation and deletion of the gene, for example, a pluripotent cell having an ability to form a germ cell, a pluripotent cell having no impairment of an immune function, or a pluripotent cell having neither mutation nor deletion of a gene involved in formation of a particular tissue or organ or a gene encoding a particular protein.

In the additional pluripotent cell, a desired gene may be modified. In this case, when a pluripotent cell having a mutation or deletion of a gene involved in germ cell formation is used as the pluripotent cell having a mutation or deletion of a particular gene and lacking a function involving the gene, a germ cell formed in the obtained chimeric animal is derived from the cell where the desired gene has been modified. Therefore, a progeny individual obtained from the chimeric animal is a transgenic animal where the desired gene has been modified. The modification of the desired gene may be introduction of a foreign gene originally absent in the pluripotent cell or may be knockout of an endogenous gene originally present in the pluripotent cell. Examples of the foreign gene include a gene derived from an animal of a different species in terms of zootaxy. The modification of the desired gene can be performed by a method known in the art.

Furthermore, the additional pluripotent cell other than the pluripotent cell having a mutation or deletion of a particular gene and lacking a function involving the gene may be a fusion cell between the pluripotent cell having a mutation or deletion of a particular gene and lacking a function involving the gene and another cell, or may be a pluripotent cell-like cell obtained by transforming a tissue stem cell (visceral organ-specific stem cell or somatic stem cell) or differentiated somatic cell of an animal by means known in the art. The tissue stem cell refers to a cell that is present in a mature animal tissue and is capable of differentiating into the tissue or other tissue cells and forming the tissue. Examples thereof include brain stem cells, liver stem cells, pancreas stem cells, kidney stem cells, heart stem cells, blood vessel stem cells, hematopoietic stem cells, bone marrow stem cells, neural stem cells, retinal stem cells, epidermal stem cells, hair follicle stem cells, and mesenchymal stem cells. These stem cells have their respective specific surface antigens and can be isolated by using antibodies specific to the antigens or by fluorescently labeling the antibodies for use and utilizing a cell sorter or the like. Alternatively, the stem cells can also be isolated as SP (side population) cells by utilizing the abilities of the stem cells to efflux a dye Hoechst 33342. A method for isolating the stem cells is well known by those skilled in the art. The isolation and differentiation potency of these tissue stem cells have been described in Experimental Medicine, Vol. 18, No. 4, 2000, p. 420-460, Experimental Medicine, Vol. 19, No. 3, 2001, 326-369, Experimental Medicine, Vol. 19, No. 15, 2001, Experimental Medicine, Vol. 21, No. 8, 2003, etc. According to the descriptions of these documents, the stem cells can be isolated, or a desired stem cell can be selected. It has been demonstrated that a fusion cell between an ES cell (pluripotent cell) and a differentiated cell, when injected into a host embryo and developed, contributes to an individual and is capable of functioning (Tada et al., Dev Dyn. 227, 504-510, 2003).

Transformed (transdifferentiated) pluripotent cells can be obtained by coculturing these stem cells or differentiated somatic cells with another pluripotent cell such as an ES cell (Andrew et al., Nature 430, 350-356, 2004; Ying et al., Nature 416, 545-548, 2002; and Terada et al., Nature 430, 542-545, 2002).

A pluripotent cell from a reproductive organ-derived cell can be established by coculturing a germ cell-derived cell with a pluripotent cell such as an ES cell. For example, a pluripotent cell such as an androgenetic embryo-derived ES cell or a pluripotent cell such as a gynogenetic embryo-derived ES cell can be established by coculturing an androgenetic embryo or gynogenetic embryo with a pluripotent cell. The coculture may be performed in a container for culture such as a dish for culture. In this coculture, appropriate feeder cells may be seeded onto the culture container. The androgenetic embryo can be constructed by removing a female pronucleus from a fertilized egg and transplanting thereinto a male pronucleus collected from another fertilized egg, while the gynogenetic embryo can be constructed by removing a male pronucleus from a fertilized egg and transplanting thereinto a female pronucleus collected from another fertilized egg. That is to say, the present invention also encompasses a method for establishing an androgenetic embryo- or gynogenetic embryo-derived ES cell by coculturing an androgenetic embryo or gynogenetic embryo with a pluripotent cell such as an ES cell.

The pluripotent cell having a mutation or deletion of a particular gene and lacking a function involving the gene, which is injected into a host embryo of an animal in the chimeric animal production, may be used as the another pluripotent cell cocultured.

The cell fusion can be performed by a method known in the art such as electroporation or a PEG method. A diploid pluripotency-like cell is obtained by transformation, and a tetraploid pluripotency cell-like cell is obtained by fusion. In this procedure, either of the cells may be converted into a microcell by treatment with colcemide such as colchicine or may be treated with mitomycin C or ultraviolet rays for heterogeneous fusion. The other cell may be used in the fusion after the removal of the mitochondrial DNA by a method known in the art.

When the fusion cell with the pluripotent cell having a mutation or deletion of a particular gene and lacking a function involving the gene is used as the additional pluripotent cell other than the pluripotent cell having a mutation or deletion of a particular gene and lacking a function involving the gene and when a transformed pluripotent cell-like cell obtained from a tissue stem cell or somatic cell by coculture with the pluripotent cell having a mutation or deletion of a particular gene and lacking a function involving the gene and is used as the additional pluripotent cell, the pluripotent cell having a mutation or deletion of a particular gene and lacking a function involving the gene is contained in the prepared fusion cell or pluripotency-like cell. Even in this case, the pluripotent cell may be injected simultaneously with the additional pluripotent cell into a host embryo in the chimeric animal production. In addition, a blastocyst-stage embryo-derived pluripotent cell, for example, an ES-like cell, which is obtained as a result of fusion, activation, and culture of a cell (e.g., a fusion cell comprising a cell, chromosome, or the like of a different species introduced therein) having the function involving the particular gene with a unfertilized egg or enucleated fertilized egg, may be injected into a host embryo, as the additional pluripotent cell other than the pluripotent cell having a mutation or deletion of a particular gene and lacking a function involving the gene. As described above, the case where the pluripotent cell having a mutation or deletion of a particular gene and lacking a function involving the gene, which is used for preparing the fusion cell or the pluripotent cell-like cell, is directly injected simultaneously therewith into a host embryo, is also applicable to the phrase "injecting into a host embryo of an animal, two or more kinds of cells comprising a pluripotent cell having a mutation or deletion of a particular gene and lacking a function involving the gene and an additional pluripotent cell other than the pluripotent cell".

The tissue stem cell or somatic cell used may be of the same or a different species in terms of zootaxy as (from) the pluripotent cell having a mutation or deletion of a particular gene and lacking a function involving the gene.

When the pluripotent cell having a mutation or deletion of a particular gene and lacking a function involving the gene has a mutation or deletion of a gene involved in germ cell formation, for example, a germ cell can be formed from a cell other than a cell derived from the pluripotent cell having a mutation or deletion of a particular gene and lacking a function involving the gene. Alternatively, when the pluripotent cell having a mutation or deletion of a particular gene and lacking a function involving the gene has a mutation or deletion of a gene involved in the immune system, for example, a cell other than a cell derived from the pluripotent cell having a mutation or deletion of a particular gene and lacking a function involving the gene functions as an immunocompetent cell that performs antibody production, and so on. Or otherwise, a cell other than a cell derived from the pluripotent cell having a mutation or deletion of a particular gene and lacking a function involving the gene produces a particular substance or forms a particular organ or tissue in a chimeric animal because during the chimeric individual formation, the cell other than a cell derived from the pluripotent cell having a mutation or deletion of a particular gene and lacking a function involving the gene is never attacked or eradicated by the immunological mechanism of the cell having a mutation or deletion of a gene involved in the immune system.

A pluripotent cell having a mutation or deletion of a gene involved in germ cell formation and being incapable of forming a germ cell is, for example, a C-kit mutant animal (W gene mutant animal)-derived cell. The C-kit is one of receptor-type tyrosine kinases mapped to the W locus and is involved in infertility and so on. Examples of the C-kit mutant animal include a W^{v}/W^{v} mouse. The W^{v}/W^{v} mouse is a hermaphroditic mouse incapable of forming a germ cell (sterile/infertile). The W^{v}/W^{v} mouse, one of C-kit mutant animals, can be obtained by a method described in, for example, Example 1 below. Other C-kit mutant animals can also be constructed according to the production method for the W^{v}/W^{v} mouse. In this case, the pluripotent cell has a mutation or deletion of a gene involved in germ cell formation and lacks the function of the germ cell formation. A chimeric animal comprising a pluripotent cell capable of forming a germ cell, which is capable of being transmitted to the germ line, can be obtained by mixing this pluripotent cell having a mutation or deletion of a gene involved in germ cell formation with an additional pluripotent cell of the same or a different species as (from) the pluripotent cell, which is capable of forming a germ cell, and injecting the cells into an early embryo of an animal.

The cell of the same or a different species as (from) the pluripotent cell having a mutation or deletion of a gene involved in germ cell formation and being incapable of forming a germ cell is selected from the group consisting of a mouse, a rat, a pig, cattle, a horse, a sheep, a goat, a monkey, and a human.

Examples of the additional pluripotent cell injected into a host embryo simultaneously with the pluripotent cell having a mutation or deletion of a gene involved in germ cell formation and being incapable of forming a germ cell include a genetically modified cell. Examples of the genetic modification include introduction of a foreign gene and knockout of an endogenous gene. A chimeric animal comprising the genetically modified cell capable of being transmitted to the germ line can be produced by injecting these cells into a host embryo of an animal. In the chimeric animal thus obtained, a germ cell is formed from the genetically modified cell. Moreover, a genetically modified animal is produced as a transgenic animal by obtaining progeny of the chimeric animal. The additional pluripotent cell injected together therewith into a host embryo may be of a different species in terms of zootaxy from the pluripotent cell having a mutation or deletion of a gene involved in germ cell formation and being incapable of forming a germ cell. In this case, the obtained chimeric animal forms a germ cell of an animal of the different species. The germ cell of the animal of the different species has a chromosome of the animal of the different species. Therefore, a stem cell of an organ or tissue having the chromosome of the animal of the different species or the organ or tissue itself is obtained from the obtained animal. Alternatively, a substance such as a protein of the animal of the different species can be obtained from a somatic cell having the chromosome of the animal of the different species in the obtained animal.

Examples of a pluripotent cell having a mutation or deletion of a gene involved in the immune system and having an impairment of immune function include a pluripotent cell derived from an immunodeficient animal such as a nude mouse (nu/nu mouse) or scid mouse. In this context, the phrase "having an impairment of immune function" refers to incapability of forming immunocompetent cells such as T cells, B cells, NK cells, and dendritic cells, or incapability of producing antibodies and a loss or reduction of the function of eradicating different species-derived cells, different species-derived tissues, different species-derived substances, and the like.

The nude mouse is a congenitally athymic mouse in which a nu gene, a recessive gene on the chromosome 11, is homozygous. Specifically, the pluripotent cell derived from the nude mouse is a pluripotent cell homozygous for the nu gene. The scid mouse refers to a mouse exhibiting a disease state similar to a human severe combined immune deficiency disease (SCID). The scid mouse, which has a homozygous scid gene (recessive gene), includes NOD/scid. Specifically, the pluripotent cell derived from the scid mouse is a pluripotent cell homozygous for the scid gene. The immunodeficient mouse such as a nude mouse or scid mouse as well as a lymphocyte-deficient mouse having a deletion of a RAG-1 gene and/or a RAG-2 gene (Chen et al., Proc. Natl. Acad, Sci. USA 90, 4528-4532, 1993) can be obtained from commercially available products or according to the descriptions of documents.

A chimeric animal can be produced by injecting into a host embryo of an animal, such a pluripotent cell having a mutation or deletion of a gene involved in the immune system and having an impairment of immune function and an additional pluripotent cell of a different species in terms of zootaxy from the pluripotent cell having a mutation or deletion of a gene involved in the immune system and having an impairment of immune function, wherein the pluripotent cell having a mutation or deletion of a gene involved in the immune system and having an impairment of immune function and the cell of a different species contribute to formation of the chimeric individual. A pluripotent cell derived from an animal such as a mouse, a rat, a pig, cattle, a horse, a sheep, a goat, or a monkey can be used as the pluripotent cell having a mutation or deletion of a gene involved in the immune system and having an impairment of immune function. A chimeric mouse contains a human antibody gene, for example, when produced by using an immunodeficient mouse-derived pluripotent cell and a pluripotent cell containing a gene encoding a human antibody. The chimeric mouse can be allowed to produce a human antibody by administering an antigen to the chimeric mouse. Further examples of the pluripotent cell having a mutation or deletion of a gene involved in the immune system and having an impairment of immune function include a pluripotent cell lacking an ability to form lymphocytes such as T cells and B cells. Examples thereof include a pluripotent cell derived from an animal having a deletion of a RAG-1 gene or RAG-2 gene involved in gene rearrangement. Such pluripotent cells also lack an immune function and, as in the use of the immunodeficient animal-derived ES cell, can be mixed with an ES cell derived from an animal of a different species to produce a chimeric animal.

Further examples of the additional pluripotent cell other than the pluripotent cell having a mutation or deletion of a particular gene and lacking a function involving the gene include a pluripotent cell having a mutation or deletion of a gene involved in formation of a particular tissue or organ or a gene encoding a particular protein and lacking an ability to form the tissue or organ or an ability to produce the protein. The gene involved in formation of a particular tissue or organ is not limited, and examples thereof include a gene involved in formation of a particular visceral organ. Likewise, the gene encoding a particular protein is not limited, and examples thereof include a gene encoding a physiologically active substance (e.g., a cytokine), immunoglobulin, or the like. Such a pluripotent cell having a mutation or deletion of a gene involved in formation of a particular tissue or organ or a gene encoding a particular protein and lacking an ability to form the tissue or organ or an ability to produce the protein is used together with an additional pluripotent cell free from the mutation and deletion of the gene to produce a chimeric animal. In the chimeric animal thus obtained, a tissue or organ derived from the additional pluripotent cell is formed, or a protein derived from the addition pluripotent cell is produced. When the additional pluripotent cell is of a different species in terms of zootaxy from the pluripotent cell having a mutation or deletion of a particular gene and lacking a function involving the gene, a tissue or organ of the different species is formed in the chimeric animal, or a protein of the different species is produced therein. The mutation or deletion of a particular gene can be performed by a genetic engineering technique known in the art.

Alternatively, a pluripotent cell having mutations or deletions of several genes and lacking several functions of the genes can also be used. Specifically, a pluripotent cell can be used in which the genes mutated or deleted are one or more genes selected from the group consisting of (a) a gene involved in germ cell formation, (b) a gene involved in the immune system, and (c) a gene involved in formation of a particular tissue or organ or a gene encoding a particular protein. For example, a pluripotent cell derived from a mouse homozygous for both a W^{v} gene and a nu gene, that is, a mouse that is incapable of form a germ cell and is immunodeficient, can be used.

Furthermore, a pluripotent cell incapable of forming a germ cell, which is obtained from interspecific crossing, can also be used as the pluripotent cell having mutations or deletions of several genes and lacking functions involving the genes. The pluripotent cell incapable of forming a germ cell can be obtained from an early embryo incapable of forming a germ cell, and the early embryo, for example, a fertilized embryo, incapable of forming a germ cell is constructed by utilizing germ cell aplasia caused by genetic factors. Specifically, an interspecific F1 (first filial generation) hybrid is known to exhibit sterility and lack germ cells, and the early embryo is constructed by utilizing this genetic factor. For example, when a mouse is used, an interspecific hybrid fertilized embryo is obtained from natural mating between an experimental mouse (Mus musculus domesticus) and a mouse in the relationship of a sibling but different species therewith. In this mating, it is desirable to obtain an ovum from an experimental female mouse having an excellent fecundity. Specifically, it is desirable to obtain an interspecific hybrid fertilized embryo from a natural mating between the experimental female mouse and a male mouse in the relationship of a sibling but different species therewith. Particularly, many fertilized embryos can be obtained by mating an experimental female mouse at the early stage from 3 weeks to 4 weeks of age after the induction of superovulation by hormone treatment. Furthermore, interspecific hybrid embryos obtained by *in-vitro* fertilization between ova collected from the experimental female mouse at the early stage where superovulation has been induced and sperms of a mouse of a different species can be obtained efficiently in large amounts. C57BL/6 or the like can be employed as the experimental mouse.

On the other hand, Mus spretus, Mus caroli, Mus pahari, or the like can be used as the mouse of a different species serving as a mating partner. Mus spretus, for which information on *in-vitro* fertilization has been accumulated relatively, is preferable, and the combination of a C57BL/6 female mouse with a Mus spretus male mouse that can stably produce interspecific hybrid embryos is preferable. Although the mouse is illustrated above, the pluripotent cell of the present invention is not limited to the mouse and encompasses pluripotent cells of all animals. In other animals, the pluripotent cell incapable of forming a germ cell, which is obtained from interspecific crossing, can be obtained by the same procedures as in the mouse. The pluripotent cell incapable of forming a germ cell, which is obtained from interspecific crossing, can also be obtained according to the descriptions of the pamphlet of International Publication of WO 03/071869.

As described above, a chimeric animal can be formed by injecting two or more kinds of cells into a host embryo (early embryo such as a blastocyst) of an animal and transplanting the early embryo into the uterus of a foster parent. Alternatively, a chimeric animal comprising a gene of a desired pluripotent cell transmitted to the germ cell can be produced by a tetraploid rescue method. The tetraploid rescue method is a method for producing a chimeric animal comprising a pluripotent cell transmitted to the germ line, which utilizes a mechanism in which as a result of injection of pluripotent cells such as ES cells into tetraploid fertilized eggs, tetraploid cells develop into placenta while only pluripotent cells such as ES cells develop into individuals (Nagy A et al., Development 110, 815-821 (1990)). When a chimeric animal is produced by the tetraploid rescue method, it is desirable to adapt the mitochondrial DNA of a tetraploid fertilized egg or ES cell to nuclear DNA in order to prevent a born animal from dying of respiratory disorder. A method for adapting the mitochondrial DNA to nuclear DNA in the tetraploid rescue method will be described later.

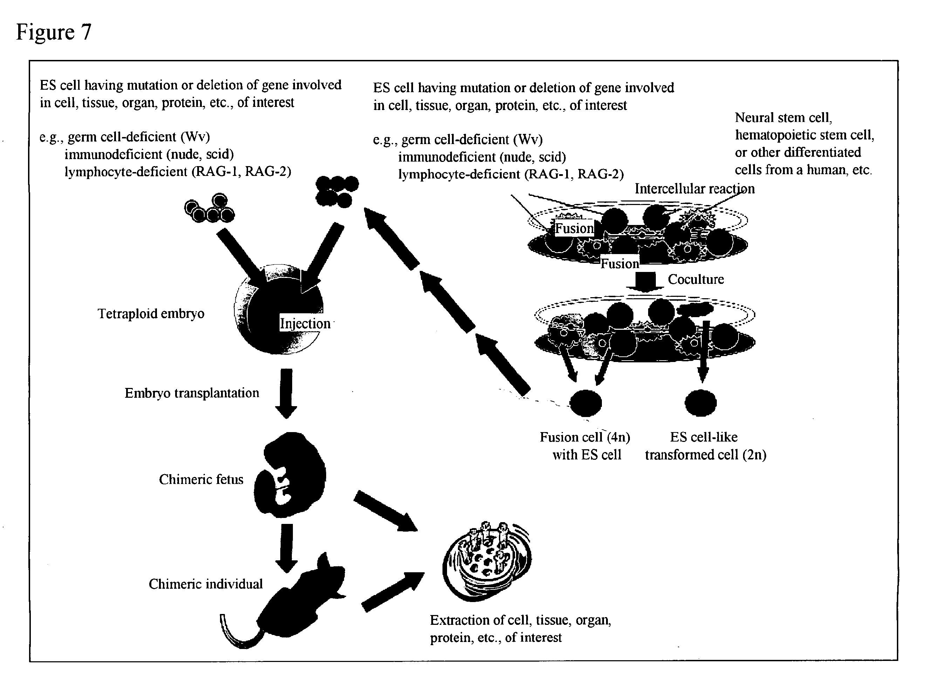

The outline of a method for producing a chimeric animal according to the method of the present invention is shown in Figure 7.

As described above, the production of a chimeric animal using two or more kinds of cells comprising a pluripotent cell having a mutation or deletion of a particular gene and lacking a function involving the gene and an additional pluripotent cell having the function has the following uses:
(1) Production of a germ cell derived from a genetically modified animal
(2) Production of a genetically modified animal
(3) Production of a stem cell derived from an animal of a different species
(4) Production of a cell, tissue, or organ derived from an animal of a different species
(5) Production of a germ cell derived from an animal of a different species
(6) Production of an animal of a different species
(7) Production of a chimeric tissue or organ containing a cell derived from an animal of a different species
(8) Production of a substance of interest by a somatic cell of a different species

Aside from this, a visceral organ stem cell or reproductive organ stem cell, when transplanted into a recipient (here, an immunodeficient mouse), adheres thereto but does not proliferate or does not differentiate into a cell of interest in many cases, from the viewpoint of regenerative medicine. This seems to be because a cellular environment surrounding the cell of a different species differs significantly from the original environment at cellular and molecular levels. On the other hand, a chimeric visceral organ produced by the method of the present invention is considered to contain a mixture of a cell of a different species, a mouse cell, and various hybrid cells. This situation is probably advantageous for the proliferation and function of a stem cell of a different species or a hybrid cell having a chromosome or gene of interest. Furthermore, it is probably advantageous for the transplantation of a visceral organ stem cell of a different, species into such a chimeric individual. The method of the present invention uses a mouse ES cell and can therefore easily achieve a genetic modification even when a substance (gene) necessary for the proliferation and differentiation of a stem cell of a different species is revealed in the future.

Knockout clone pigs deficient in an enzyme involved in antigenicity of acute rejection have been produced for the purpose of being used in visceral organ transplantation. If a chimeric animal is produced by further producing immunodeficient pigs from these pigs and constructing fusion cells between human cells and pig cells by the method of the present invention, those closer to human visceral organs are produced.

The present invention also encompasses a chimeric animal and progeny thereof obtained by the method described above.

The present invention further encompasses the pluripotent cell itself having a mutation or deletion of a particular gene and lacking a function involving the gene. A cell included in the pluripotent cell is the same as above.

Hereinafter, the production method and uses of the pluripotent cell of the present invention will be described in detail by taking a W^{v}/W^{v} mouse embryo-derived ES cell as an example. However, the origin of the pluripotent cell is not limited to the W^{v}/W^{v} mouse and encompasses an immunodeficient mouse such as a nu/nu mouse or scid mouse, a lymphocyte-deficient mouse, other mice, and other animals such as rats, cattle, horses, sheep, goats, and monkeys. Likewise, the pluripotent cell is not limited to the ES cell and encompasses an EG cell and a GS cell.

ES cells can be established by *culturing in vitro* inner cell masses of blastocysts at the mammalian developmental stage (Evans, M.J. et al., Nature, 292: 154-156, 1981; and Thomson, J. A., Science, 282: 1145-1147, 1998).

To form chimeric mice from ES cells in embryos, the established ES cells must have a high proliferating ability. The proliferating ability includes both an *in-vitro* proliferating ability and a proliferating ability in embryos.

Specifically, the ES cell can be established by culturing *in vitro* a cell of an inner cell mass of a blastocyst, and in this procedure, coculture with an additional ES cell or tetraploid embryo can establish an ES cell having a high undifferentiated form, differentiation potency, and proliferating ability. Moreover, the rate of establishment is improved. The ES cell used here may be any cell that can be distinguished and separated from an ES cell of interest. It is desirable to use a W^{v}/W^{v} mouse embryo-derived ES cell incapable of forming a germ cell in order to establish an ES cell for the purpose of producing a germ line chimera. For example, the rate of establishment from KK mice is 0% without coculture with the W^{v}/W^{v} mouse embryo-derived ES cell, whereas the rate is increased to 50% with the coculture. The coculture may be performed in a container for culture such as a dish for culture. In this coculture, appropriate feeder cells may be seeded onto the culture container. Furthermore, the coculture may be performed in an early embryo constituting an inner cell mass (ICM). In this case, several cells to be cocultured may be injected into an early embryo by use of a micromanipulator or the like and then cultured. After coculture for a fixed period, the cocultured cells are collected, and a tetraploid may be injected into an early embryo such as an embryo (tetraploid) for producing a chimeric animal. The early embryo used can be reused in coculture. Alternatively, a tetraploid may be used as the early embryo. The method described above can efficiently establish ES cells even from reconstructed embryos such as nuclear-transplanted embryos and male embryos, from which ES cells are difficult to establish. The reconstructed embryos cocultured with the W^{v}/W^{v} mouse embryo-derived ES cell or tetraploid embryo can also be used directly in the production of a chimeric animal.

As described later, several ES cells to be cocultured are injected into an early embryo for producing a chimeric animal, and a chimeric animal may be produced directly therefrom.

The establishment of the additional ES cell using the W^{v}/W^{v} mouse embryo-derived ES cell of the present invention may be performed by a method described in, for example, Example 5 below.

The enhanced proliferating ability of the desired ES cell during culture and the improved proliferation of the ES cell after transplantation into an embryo are accomplished by coculturing the W^{v}/W^{v} mouse embryo-derived ES cell of the present invention with the additional ES cell, for example, an ES cell that has received a desired genetic modification such as introduction of a foreign gene or knockout of a particular gene, and simultaneously injecting them into an early embryo. As a result, a chimeric mouse comprising a gene of the ES cell transmitted to the germ line can be obtained. The additional ES cell cocultured with the W^{v}/W^{v} mouse embryo-derived ES cell of the present invention is also maintained morphologically favorably. Excellent ES cell colonies have a round and clear outline in which the cells proliferate in a raised pattern. By contrast, poor ES cell colonies are oblate and misshapen.

On the other hand, the W^{v}/W^{v} mouse embryo-derived ES cell does not form a germ cell and is therefore never transmitted to the germ line. That is to say, when a chimeric animal is obtained by the method of the present invention, a sperm or ovum of the obtained chimeric animal is derived from the additional ES cell other than the W^{v}/W^{v} mouse embryo-derived ES cell, and a chromosome derived from the additional ES cell is transmitted to next-generation newborns without exception. In this case, the desired ES cell that can be used may be derived from any mouse strains and is desirably a C57BL/6 strain- or 129 strain-derived ES cell, particularly desirably a C57/BL6-derived ES cell. Alternatively, a commercially available ES cell may be used here.

Alternatively, an ES cell enhanced in its proliferating ability during culture and in its proliferating ability after transplantation to an embryo can also be established by using a medium supplemented with a condition medium of the W^{v}/W^{v} mouse embryo-derived ES cell in the establishment of the additional ES cell, instead of coculture with the W^{v}/W^{v} mouse embryo-derived ES cell. The condition medium can be prepared according to the descriptions of, for example, Martin et al., Dev Biol 121, 20-28, 1987.

In this procedure, it is desirable that the W^{v}/W^{v} mouse embryo-derived ES cell cocultured with the desired ES cell should have a proliferating ability that falls within a fixed range. If the ES cell has a low proliferating ability, the proliferating ability of the ES cell cocultured therewith cannot be improved. On the other hand, if the ES cell has a too high proliferating ability, all the ES cells are replaced by the W^{v}/W^{v} mouse embryo-derived ES cell. When the ES cell maintains an undifferentiated form and differentiation potency and has a too high proliferating ability, its proliferating ability can be adjusted by treatment with mitomycin C, ultraviolet rays, rhodamine, or the like.

When a chimeric mouse is produced by using ES cells, their proliferating abilities are generally reduced with increases in the passage number of the ES cells, making transmission to the germ line difficult. Therefore, it is usually necessary to use ES with a passage number not more than 10. On the other hand, long-term subcultured ES cells, for example, ES cells subcultured to the 12th or more generation or 20th or more generation, if used after coculture with the W^{v}/W^{v} mouse embryo-derived ES cell, can also be used because their proliferation and so on are supported by the W^{v}/W^{v} mouse embryo-derived ES cell.

A chimeric mouse can be formed efficiently by injecting the W^{v}/W^{v} mouse embryo-derived ES cell of the present invention together with a desired additional ES cell into an early embryo such as a blastocyst and transplanting the early embryo into the uterus of a foster parent. Alternatively, a chimeric mouse comprising a gene of a desired ES cell transmitted to the germ cell can be produced by a tetraploid rescue method. The tetraploid rescue method is a method for producing a chimeric mouse comprising an ES cell transmitted to the germ line, which utilizes a mechanism in which as a result of injection of ES cells into tetraploid fertilized eggs, tetraploid cells develop into placenta while only ES cells develop into individuals (Nagy A et al., Development 110, 815-821 (1990)). When a chimeric mouse is produced by the tetraploid rescue method, it is desirable to adapt the mitochondrial DNA of a tetraploid fertilized egg or an ES cell to be mixed with the W^{v}/W^{v} mouse embryo-derived ES cell to nuclear DNA in order to prevent a bom mouse from dying of respiratory disorder. However, when the cell used is a hybrid cell, for example, a hybrid cell from two kinds of inbred animals, the mitochondrial DNA is not necessarily required to be adapted to nuclear DNA because a born mouse does not present the problem of respiratory disorder. For example, the W^{v}/W^{v} mouse used in the present invention is an inbred mouse. Therefore, the W^{v}/W^{v} mouse embryo-derived ES cell does not necessarily require adapting the mitochondrial DNA to nuclear DNA. To adapt the mitochondrial DNA to nuclear DNA, the mitochondrial DNA of the tetraploid fertilized egg or the ES cell to be mixed with the W^{v}/W^{v} mouse embryo-derived ES cell may be substituted with the mitochondrial DNA of a wild-type mouse, specifically, as follows (the pamphlet of International Publication of WO 03/071869):
A mitochondrial DNA-substituted ES cell derived from a mouse embryo can be obtained by a method known in the art. For example, the substitution of mitochondrial DNA of an inbred mouse is performed by a back-crossing method, nucleus substitution method, or the like, to produce an inbred mouse comprising a desired type of mitochondrial DNA substituted for the original mitochondrial DNA. Then, an ES cell may be established from the mouse. A C57BL/6 inbred mouse can repeatedly be crossed 12 or more times with a Mus musculus musculus wild-type mouse by the back-crossing method to thereby produce a B6-mtMus inbred mouse in which the mitochondrial DNA of the C57BL/6 inbred mouse has been substituted with the Mus musculus musculus-type mitochondrial DNA of the wild-type mouse. From this mouse, an ES cell comprising the mitochondrial DNA substituted with the adapted mouse Mus musculus musculus-type DNA can be established.

On the other hand, the pronucleus substitution method is performed according to a standard method (McGrath J & Solter D, J. Exp. Zool, 228, 355-362 (1983)) to transplant the male and female pronuclei of an pronuclear-stage fertilized egg of an mdx inbred mouse into an enucleated pronuclear-stage fertilized egg having a Mus musculus musculus-type mitochondrion of a wild-type mouse. After nuclear fusion by electric pulses and subsequent culture, the egg can be transplanted into a mouse oviduct for ontogenesis to thereby produce an mdx-mtMus inbred mouse comprising the mitochondrial DNA substituted with the Mus musculus musculus-type mitochondrial DNA. Similarly, an ES cell comprising the mitochondrial DNA substituted with wild-type mitochondrial DNA can be established from this mouse.

To establish an ES cell, a blastocyst is collected from the inbred mouse thus produced, for example, B6-mtMus or mdx-mtMus, and an ES cell can be established by a standard method (Evans MJ and Kaufman MK, Nature 292, 154-156 (1981)).

When mitochondrial DNA substitution is conducted on the W^{v}/W^{v} mouse embryo-derived ES cell, male B6-W^{v}/+ is mated with female B6-mtMus to obtain a B6-W^{v}/+-mtMus mouse. Then, female (♀) B6-W^{v}/+-mtMus is mated with a male DBA mouse to obtain a BDF1-W^{v}/+-mtMus mouse. Subsequently, male and female BDF1-W^{v}/+-mtMUS mice may be mated. As described above, the W^{v}/W^{v} mouse embryo-derived ES cell of the present invention can be used in the establishment of an additional ES cell, in the preparation of an ES cell having a proliferating ability sufficient for chimeric mouse production by coculture with a desired genetically modified ES cell, and in the production of a chimeric mouse by simultaneous injection with the desired ES cell into a blastocyst. The use of the W^{v}/W^{v} mouse embryo-derived ES cell at these steps may be use only at each step or may be consecutive use at two or more steps. For example, the W^{v}/W^{v} mouse embryo-derived ES cell is cocultured with a desired additional genetically modified ES cell, and the W^{v}/W^{v} mouse embryo-derived ES cell and the additional ES cell may be injected directly for chimeric mouse production into a blastocyst.

When an ES cell is genetically modified for constructing a knockout mouse or the like, the proliferating ability of the ES cell is often reduced. The W^{v}/W^{v} mouse embryo-derived ES cell of the present invention can also improve the proliferating ability and so on of such a genetically modified ES cell. The W^{v}/W^{v} mouse embryo-derived ES cell of the present invention can be used particularly preferably for an ES cell that has undergone several genetic modifications.

Furthermore, an EG cell (embryonic germ cell) or GS cell in addition to the ES cell may be used as a cell that can be established with the W^{v}/W^{v} mouse embryo-derived ES cell of the present invention, as a desired genetically modified cell, and as a cell injected simultaneously therewith into a blastocyst. The EG cell is obtained by coculturing a mammalian primordial germ cell with a cytokine such as LIF or FGF2 (Matsui, Y., et al., Cell, 70: 841-847, 1992). The GS cell can be obtained from the testis of a male mouse at infancy (Johnson et al. Nature 428, 145-150, 2004).

The GS cell is established by, for example, the following procedures:
Establishment of mouse GS-like cell
   1. Coat a dish with gelatin.
   2. Enzyme Solution 1 contains 5 ml of DMEM/F12, 3 mg/ml collagenase, and 200 µg/ml DNase I.
   3. Enzyme Solution 2 contains 5 ml of DMEM/F12, 3 mg/ml collagenase, 200 µg/ml DNase I, and 3 mg/ml hyaluronidase.
   4. Cut the testis of a 10-day-old to 2-week-old mouse into pieces in the Enzyme Solution 1.
   5. Incubate at 37°C for 15 minutes.
   6. Centrifuge at 20°C for 10 minutes at 2300 rpm.
   7. Aspirate the supernatant with an aspirator and add thereto the enzyme solution of the tube II'. In this procedure, add trypsin at a concentration of 500 µg/ml.
   8. Incubate at 37°C for 30 minutes.
   9. Stop the reaction with a serum-containing medium and then centrifuge at 20°C for 10 minutes at 2300 rpm.
   10. Aspirate the supernatant with an aspirator and add thereto 5 ml of DMEM/F12 (containing serum).
   11. Filtrate with an 80-µm nylon mesh.
   12. Then filtrate with a 40-µm mesh.
   13. Culture in a DMEM/F12 or ES medium containing 5 to 20% FBS. Replace the medium once every two days (for DMEM/F 12) or everyday (for ES).

When a chimeric mouse is produced by injecting the W^{v}/W^{v} mouse-derived ES cell of the present invention together with a desired ES cell into a blastocyst, the sex ratio of born mice can be controlled. The general establishment of ES cells often produces cells having male XY-type sex chromosomes and hardly produces female XX-type cells. On the other hand, cells having female XX-type sex chromosomes can be obtained easily by the W^{v}/W^{v} mouse embryo-derived ES cell of the present invention. Both male XY-type and female XX-type cells can be selected, as required, as to the W^{v}/W^{v} mouse embryo-derived ES cell. For example, when a desired male XY-type ES cell and a male XX-type W^{v}/W^{v} mouse embryo-derived ES cell are injected into a blastocyst, all born chimeric mice are male. When a desired male XY-type ES cell and a female XX-type W^{v}/W^{v} mouse embryo-derived ES cell are injected into a blastocyst, male or female chimeric mice are born in accordance with the strength of the proliferating ability of the desired ES cell.

The sex of a possible chimeric animal obtained by the method for producing a chimeric animal and the type and origin of the obtained germ cell are as follows:

| | Embryo | ES | Chimera | Germ cell |
|---|---|---|---|---|
| 1. | Male | Male | Male | Sperm (embryo-derived + ES-derived) |
| 2. | Male | Female | Male | Sperm (embryo-derived) |
| 3. | Male | Female | Female | Egg (ES-derived + rarely, embryo-derived) |
| 4. | Female | Male | Male | Sperm (ES-derived) |
| 5. | Female | Male | Female | Egg (embryo-derived + rarely, ES-derived) |
| 6. | Female | Female | Female | EGG (embryo-derived + ES-derived) |

In the conventional methods, the above cases 2, 3, and 6 hardly occur because ES cells are usually male. Specifically, when a female chimera is born in the conventional methods, only the case 5 gives, albeit rarely, an ES cell-derived egg. Thus, the germ line transmission of the ES cell is difficult.

When the W^{v}/W^{v} mouse embryo-derived ES cell of the present invention is used, the use of the ES cell derived from the W^{v}/W^{v} mouse embryo used as the embryo in the case 5 gives eggs all derived from ES in the case that female chimeras are born. Thus, if their children are born, the ES cell is transmitted 100% to the germ line.

A chimeric mouse can be obtained at a almost 100% probability by simultaneously injecting the W^{v}/W^{v} mouse embryo-derived ES cell of the present invention in coculture with the additional ES cell and in injection with the additional ES cell into a blastocyst.

When a chimeric mouse is produced by using the W^{v}/W^{v} mouse embryo-derived ES cell of the present invention, a chimeric mouse comprising a gene of a desired ES cell transmitted to the germ line can be produced 100%. Therefore, if a born mouse dies, a desired chimeric mouse can be produced by transplanting the gonad of the mouse into another mouse.

Furthermore, the present invention encompasses a method for producing a germ cell from an androgenetic embryo- or gynogenetic embryo-derived ES cell. The androgenetic embryo- or gynogenetic embryo-derived ES cell can be established as a pluripotent cell from a reproductive organ-derived cell by coculturing a germ cell-derived cell with a pluripotent cell such as an ES cell. For example, a pluripotent cell such as an androgenetic embryo-derived ES cell or a pluripotent cell such as a gynogenetic embryo-derived ES cell can be established by coculturing an androgenetic embryo or gynogenetic embryo with a pluripotent cell. A pluripotent cell can also be established by coculturing the androgenetic embryo- or gynogenetic embryo-derived ES cell thus obtained with an additional early embryo or germ cell-derived cell.

A germ cell can also be formed from the androgenetic embryo- or gynogenetic embryo-derived ES cell. To form a germ cell, the androgenetic embryo- or gynogenetic embryo-derived ES cell may be transplanted into the reproductive organ of an animal. For example, the androgenetic embryo-derived ES cell can be transplanted into a seminiferous tubule to thereby form a sperm derived from the androgenetic embryo-derived ES cell. In this procedure, the animal into which the androgenetic embryo-derived ES cell is transplanted is desirably an animal of the same species as the source animal for the ES cell. Preferably, the ES cell is treated with mitomycin or the like for the transplantation. Similarly, the gynogenetic embryo-derived ES cell can be transplanted into an oviduct to thereby obtain an ovum derived from the gynogenetic embryo-derived ES cell. To investigate whether the finally obtained germ cell is derived from the ES cell, the state of genomic imprinting may be confirmed.

That is to say, the present invention encompasses a method for producing a germ cell by transplanting an androgenetic embryo- or gynogenetic embryo-derived pluripotent cell into the reproductive organ of an animal.

The present invention will be described in detail with reference to Examples below. However, these Examples merely provide illustrations and do not limit the present invention by any means.

### [Example 1] Establishment of WW mouse embryo-derived ES cell lines (W^{v}/W^{v} ES cell lines)

Female C57BL-W^{v}/+ mice (Japan SLC, Inc.) were subjected to natural mating with male DBA/2J (CLEA Japan, Inc.) to F1 (first filial generation) hybrids. Individuals having heterozygous W^{v} genes, that is, BDF1-W^{v}/+, were selected by hair color. Similarly, F2 hybrid heterozygous individuals (BDF2-W^{v}/+)) were obtained by natural mating between male and female BDF1-W^{v}/+ mice. Male and female BDF2-W^{v}/+ mice were subjected to natural mating. Three days after the day when vaginal plugs were confirmed, the uteruses of the individuals were perfused with an M2 medium, and 34 blastocyst-stage embryos were collected. From the obtained embryos, ES cells were established according to a standard method (Evans MJ and Kaufman MK, Nature 292, 154-156 (1981)). Specifically, the embryos obtained in advance were transferred on a one-by-one basis onto feeder cells in a 4-well plate to which an ES medium (80% (v/v) DMEM, 20% (v/v) FCS, 1% (v/v) 100 mM pyruvic acid solution (Gibco Cat. 11360-070), 1% (v/v) nonessential amino acid solution (Gibco Cat. 11140-027), 1 mM 2-mercaptoethanol (Sigma, Cat. No. M-6250), 10³ U/mL LIF) was dispensed. The embryos were cultured under conditions of 37°C and 5% CO₂-5% O₂-90% N₂. On the 7th culture day, the proliferating ICM cell masses were selected. The cells were dispersed by treatment with 0.125% trypsin/0.1 mM EDTA, then seeded onto feeder cells in a newly prepared 4-well plate, and cultured. In the second subculture, the whole wells were treated with trypsin/EDTA, and the cells were seeded onto a new 3.5-cm culture dish to obtain 17 favorably proliferating lines. The cells that could reach this point could stably proliferate thereafter and were therefore cryopreserved as cell lines.

The genomic typing of the established ES cells was conducted by a PCR-RFLP method (British Journal of Haematology 98, 1017-1025 (1997)). PCR was performed with primers 5'-AAAGAGAGGCCCTAATGTCGG-3' (SEQ ID NO: 1) and 5'-CTCGAGACTACCTCCCACC-3' (SEQ ID NO: 2) recognizing W genes, and the obtained 104-bp amplification fragment was treated with an Nsi1 restriction enzyme to assay unfragmented wild types and W^{v} types fragmented to 85 bp and 19 bp. As a result, 5 lines were W^{v}/W^{v} types. When these lines were subjected to sex determination PCR (Kay et al. Cell 77, 639-650, 1994), 2 lines were male strains and 3 lines were female strains.

### [Example 2] Establishment of 129.B6-YGFP ES cells

Female 129/sv mice (NIH) were subjected to natural mating with male C57BL/6-EGFP #60 (Y-link) (Ichida et al., J Biol Chem 26, 275, 15992-6001 (2000)). Three days after the day when vaginal plugs were confirmed, the uteruses of the individuals were perfused with an M2 medium, and 10 blastocyst-stage embryos were collected. From the obtained embryos, ES cells were established according to a standard method (Evans MJ and Kaufman MK, Nature 292, 154-156 (1981)). Specifically, the embryos obtained in advance were transferred on a one-by-one basis onto feeder cells in a 4-well plate to which an ES medium (80% (v/v) DMEM, 20% (v/v) FCS, 1% (v/v) 100 mM pyruvic acid solution (Gibco Cat. 11360-070), 1% (v/v) nonessential amino acid solution (Gibco Cat. 11140-027), 1 mM 2-mercaptoethanol (Sigma, Cat. No. M-6250), 10³ U/mL LIF) was dispensed. The embryos were cultured under conditions of 37°C and 5% CO₂-5% O₂-90% N₂. On the 7th culture day, the proliferating ICM cell masses were selected. The cells were dispersed by treatment with 0.125% trypsin/0.1 mM EDTA, then seeded onto feeder cells in a newly prepared 4-well plate, and cultured. In the second subculture, the whole wells were treated with trypsin/EDTA, and the cells were seeded onto a new 3.5-cm culture dish to obtain 5 favorably proliferating lines. The cells that could reach this point could stably proliferate thereafter and were therefore cryopreserved as cell lines.

When these lines were subjected to sex determination PCR (Kay et al. Cell 77, 639-650, 1994), 1 line was a male strain and 4 lines were female strains.

### [Example 3] Establishment of BS-neo ES cells

Female B6-mtMus mice (N15) in which the mitochondrial DNAs of C57BL/6J mice were substituted with Mus musculus musculus-type mitochondrial DNAs (Nagao et al., Genes Genet Syst 73, 21-27 (1998)) were subjected to natural mating with male C57BL/6J mice. Three days after the day when vaginal plugs were confirmed, the uteruses of the individuals were perfused with an M2 medium, and 20 blastocyst-stage embryos were collected. From the obtained embryos, ES cells were established according to a standard method (Evans MJ and Kaufman MK, Nature 292, 154-156 (1981)). Specifically, the embryos obtained in advance were transferred on a one-by-one basis onto feeder cells in a 4-well plate to which an ES medium (80% (v/v) DMEM, 20% (v/v) FCS, 1% (v/v) 100 mM pyruvic acid solution (Gibco Cat. 11360-070), 1% (v/v) nonessential amino acid solution (Gibco Cat. 11140-027), 1 mM 2-mercaptoethanol (Sigma, Cat. No. M-6250), 10³ U/mL LIF) was dispensed. The embryos were cultured under conditions of 37°C and 5% CO₂-5% O₂-90% N₂. On the 5th culture day, the proliferating ICM cell masses were selected. The cells were dispersed by treatment with 0.125% trypsin/0.1 mM EDTA, then seeded onto feeder cells in a newly prepared 4-well plate, and cultured. In the second subculture, the whole wells were treated with trypsin/EDTA, and the cells were seeded onto a new 3.5-cm culture dish to obtain 2 favorably proliferating lines. The cells that could reach this point could stably proliferate thereafter and were therefore cryopreserved as cell lines.

When these lines were subjected to sex determination PCR (Kay et al. Cell 77, 639-650, 1994), 1 line was a male strain and 1 line was a female strain.

A neo gene vector was gene-transferred to the male line of the established B6-mtMus ES cell line by a standard method, followed by gene targeting.

### [Example 4] Preparation of tetraploid blastocyst-stage embryos

ICR mice that underwent superovulation-inducing treatment were subjected to natural mating with male mice of the same strain. Forty-four to forty-six hours after chorionic gonadotropic hormone (hCG) administration, late two-cell-stage embryos were collected by an oviduct perfusion method from individuals where vaginal plugs were confirmed. The obtained late two-cell-stage embryos were subjected to fusion treatment (Goku manufactured by FUJITA; pulse conditions: 3 runs each at 18 V at 50-µsec and 999-µsec intervals) by direct current electric pulses in 0.3 M mannitol to prepare tetraploid embryos. The tetraploids were screened and cultured at 37°C for 2 days in 5% CO₂-Air in an M16 medium to obtain tetraploid blastocyst-stage embryos.

### [Example 5] Coculture with W^{v}/W^{v} ES cells

The BS-neo ES cells were damaged due to drug selection or the like after gene transfer and therefore cocultured with W^{v}/W^{v} ES cells. Specifically, 1×10⁴ BS-neo ES cells were mixed with 1×10⁴ W^{v}/W^{v} ES cells and seeded onto a 6-cm dish where feeder cells were spread. On the third day, subculture was performed in the same way as in the usual preservation of ES cells. After culture for additional 3 days, the cells were dispersed by treating 20 colonies for cloning having favorable morphologies with 0.125% trypsin/0.1 mM EDTA, then separately transferred onto feeder cells in a newly prepared 4-well plate, and cultured. On the third culture day, 3 to 5 colonies proliferating from each colony were used to conduct genomic typing by the above-described PCR-RFLP method (British Journal of Haematology 98, 1017-1025 (1997)). Of 20 lines, 7 lines were BS-neo ES cell lines. The lines having the best colony morphology and proliferation among them were cryopreserved and used for chimera production.

### [Example 6] Production of chimeric mice by tetraploid rescue method (1)

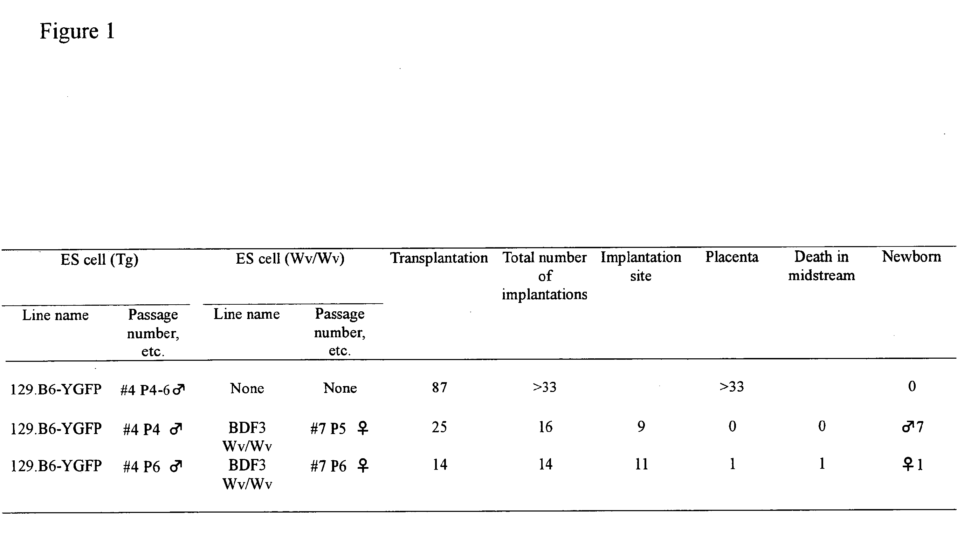

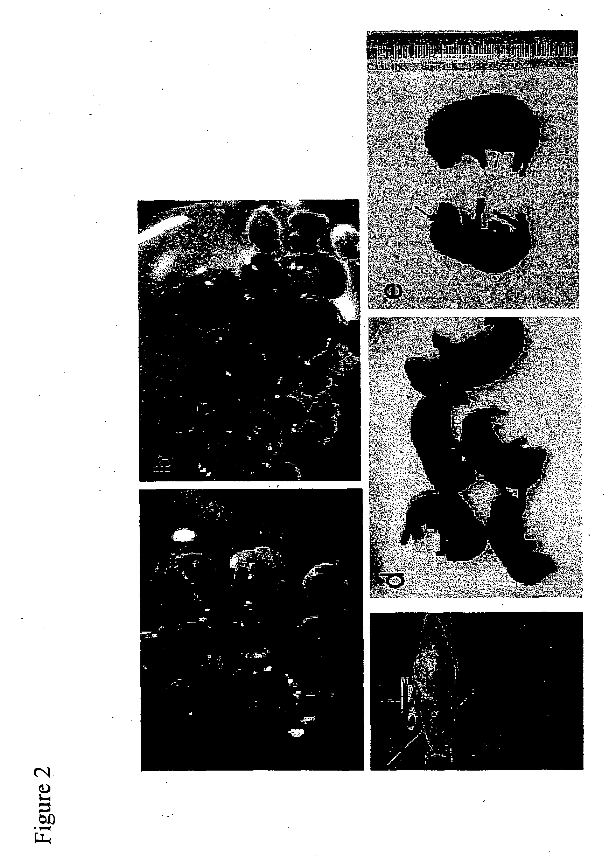

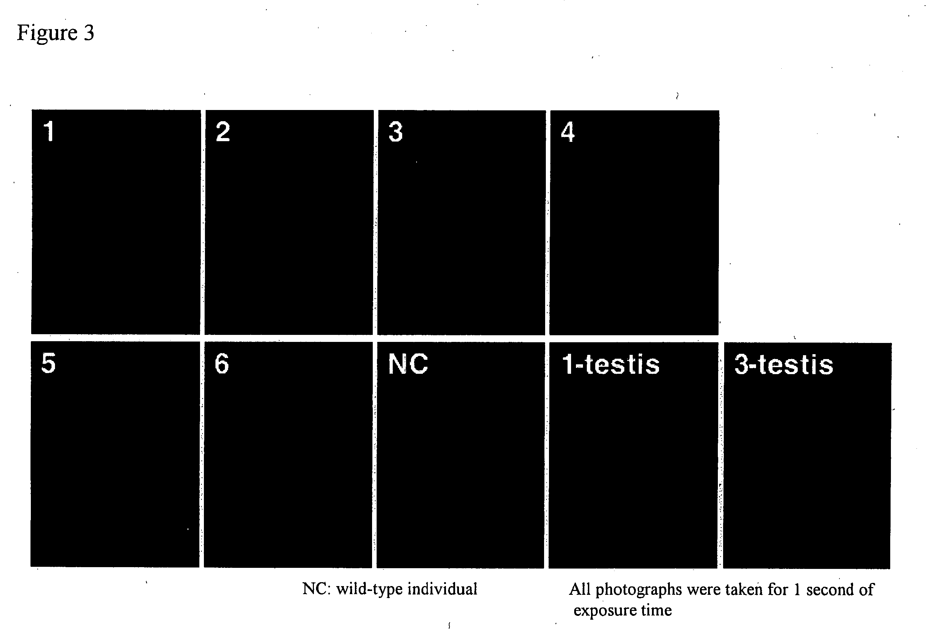

The above-described 129.B6-YGFP ES cells (male) and the BDF3 W^{v}/W^{v} ES cells (female) were injected at about 10 cells each into the tetraploid blastocyst-stage embryos obtained in Example 4 by use of a piezo-micromanipulator. Thirty-nine tetraploid blastocyst-stage embryos (tetraploids) after the injection were transplanted into the uteruses of two recipient ICR mice on the 2.5th day after the confirmation of vaginal plugs. On the eve of birth (night on the 18.5th day of pregnancy), total 8 chimeric mouse fetuses of 7 males and 1 female were taken out by cesarean section. The outline of chimeric mouse production is shown in Figure 1. In Figure 1, the upper left cells are ES cells having a mutation or deletion of a gene involved in a cell, tissue, organ, protein, or the like, of interest, such as germ cell-deficient (Wv), immunodeficient (nude, scid), and lymphocyte-deficient (RAG-1, RAG-2) cells, and the upper right cells are ES cells having a mutation or deletion of a gene involved in a cell, tissue, organ, protein, or the like, of interest, such as neural stem cells, hematopoietic stem cells, and other differentiated cells from a human, etc., and germ cell-deficient (Wv), immunodeficient (nude, scid), and lymphocyte-deficient (RAG-1, RAG-2) cells. Finally, the cell, tissue, organ, protein, or the like, of interest can be taken out of the obtained chimeric individuals. Photographs of chimeric mice are shown in Figure 2. Results of observation with a fluorescence microscope (GFP) demonstrated that the ES cells of interest (129, B6-YGFP) contribute to all the obtained chimeric fetuses. As shown in Figures 2a and 2b, when chimeric mice were produced with 129.B6-Ylink-GFP ES + tetraploid embryos without the rescue of W^{v}/W^{v} ES cells, the formation of fetuses was not observed, and only placenta or implantation sites were observed. Figure 2c is a diagram showing 129.B6-Ylink-GFP ES + W^{v}/W^{v} ES #7 + tetraploid embryo-transplanted recipient mice on the 18.5th day of pregnancy. Figure 2d is a diagram showing 129.B6-Ylink-GFP ES + W^{v}/W^{v} ES #7 + tetraploid chimeric mice immediately after cesarean section. As shown in Figure 2e, of the born individuals, two newborns exhibited abnormal morphologies such as a lack of eyeballs or ascites (arrows in Figure 2e). However, no abnormality was observed in other newborns. The results of fluorescent microscope observation of the chimeric mice (129.B6-Ylink-GFP ES + W^{v}/W^{v} ES + tetraploid chimeric mice, 8-day-old) are shown in Figure 3. Following resuscitation treatment, the mice were confirmed to actively move and then raised by foster parents.

### [Example 7] Production of chimeric mice by tetraploid rescue method (2)

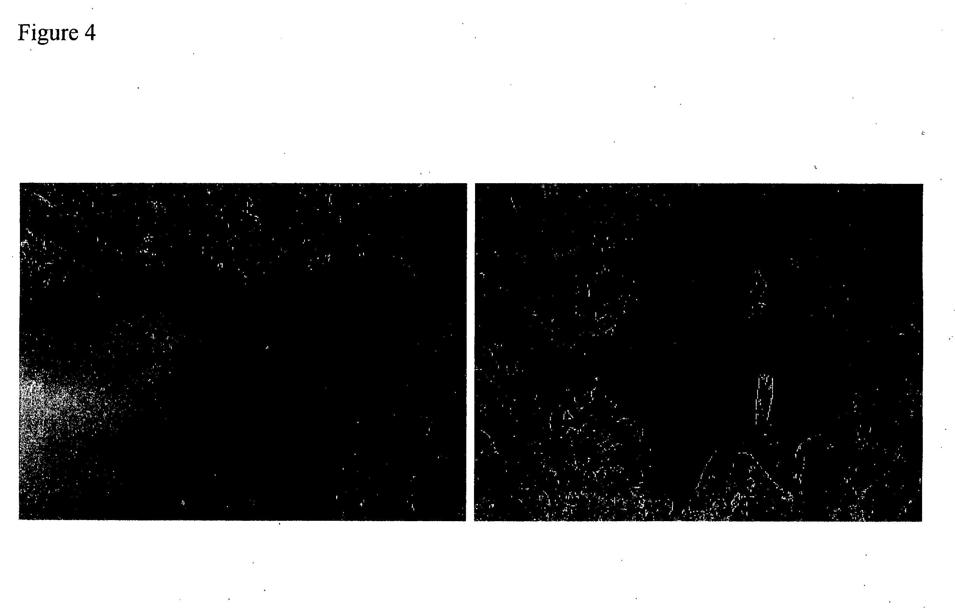

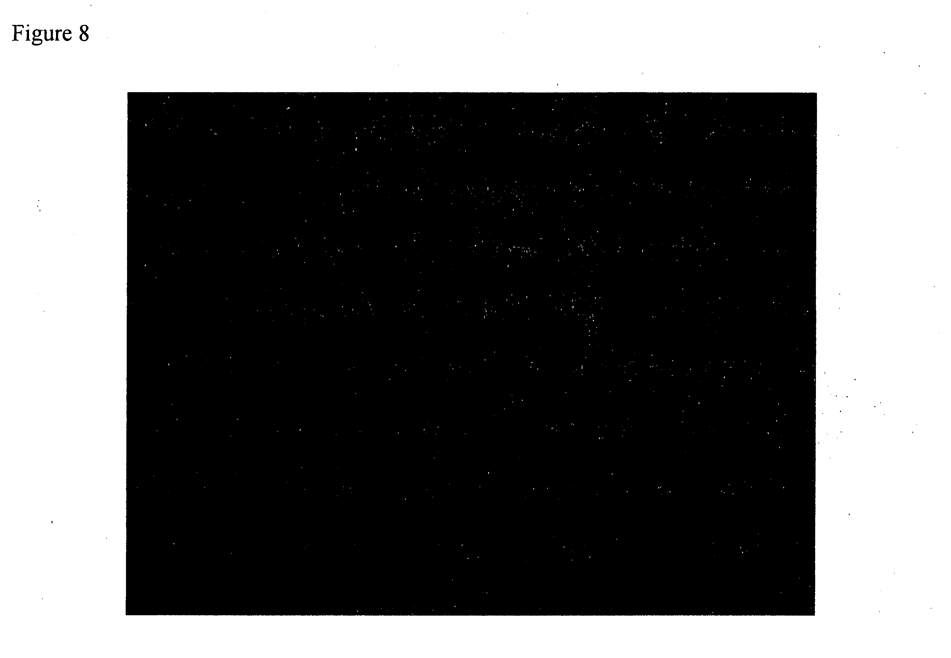

The above-described BS-neo ES cells (male) and the BDF3 W^{v}/W^{v} ES cells (male) were injected at about 10 cells each into the tetraploid blastocyst-stage embryos obtained in Example 4 by use of a piezo-micromanipulator. Twenty-nine tetraploid blastocyst-stage embryos after the injection were transplanted into the uteruses of two recipient ICR mice on the 2.5th day after the confirmation of vaginal plugs. On the eve of birth (night on the 18.5th day of pregnancy), 3 male fetuses were taken out by cesarean section or natural birth. Following resuscitation treatment, the mice were confirmed to actively move and then raised by foster parents. Hair color demonstrated that the ES cells of interest (BS-neo ES cells) contribute to one of the obtained chimeric fetuses. Photographs of the obtained chimeric mice (BS-neo ES (black color) + W^{v}/W^{v} ES (white color) + tetraploid chimeric mice, 8-day-old) are shown in Figure 4. In Figure 4, the BS-neo ES cells contribute to the individual having black hair color, and the white individuals are formed from only the W^{v}/W^{v} ES cells.

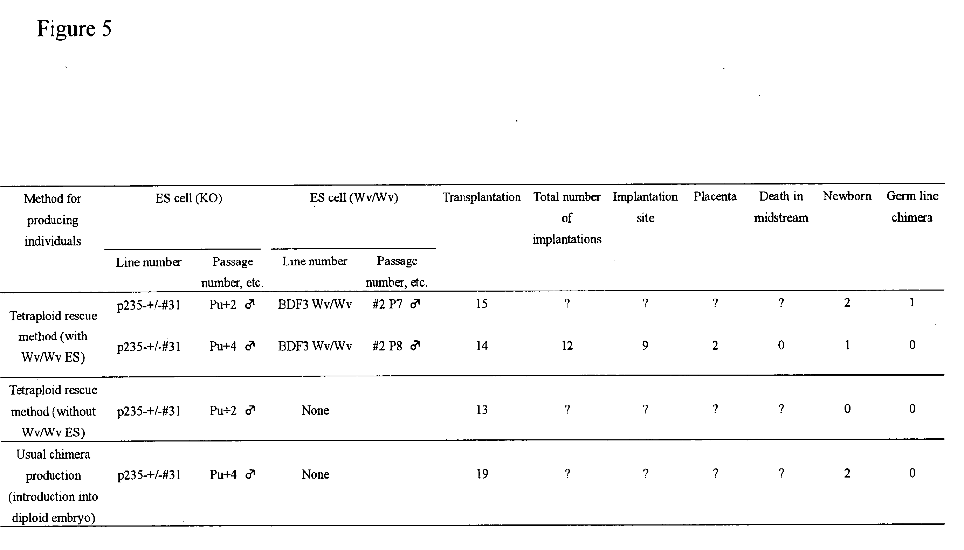

The results of a series of chimeric mouse production are shown in Figure 5.

### [Example 8] Production of chimeric mice by tetraploid rescue method (3)

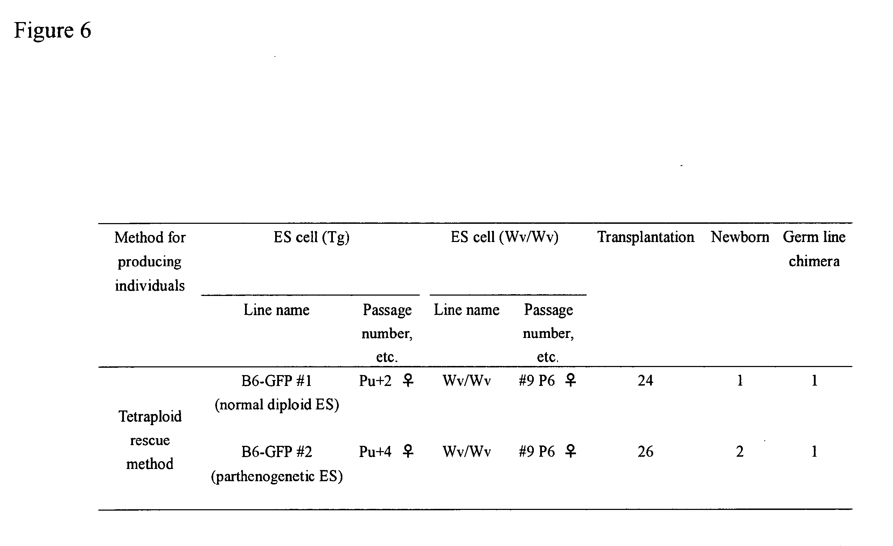

Female B6-GFP ES cells (Ichida et al., 2000) established by the same technique as in Examples 1 and 2 or B6-GFP parthenogenetic embryo-derived ES cells (B6-GFP PGES) constructed according to a standard method (Barton et al., 1987) were injected at about 10 cells each together with BDF3 W^{v}/W^{v} ES cells (female) into the tetraploid blastocyst-stage embryos obtained in Example 4 by use of a piezo-micromanipulator. Twenty-four B6-GFP ES cell chimeric embryos and twenty-six B6-GFP PGES chimeric embryos after the injection were transplanted into the uteruses of two recipient ICR mice on the 2.5th day after the confirmation of vaginal plugs to obtain one B6-GFP ES cell chimeric embryo-derived individual and two B6-GFP PGES chimeric embryo-derived individuals by natural birth. Results of observation with a fluorescent microscope (GFP) demonstrated that the GFP-ES cells of interest contribute to one B6-GFP ES cell chimeric embryo-derived individual and one B6-GFP PGES chimeric embryo-derived individual. The details of this chimeric mouse production are shown in Figure 6.

### [Example 9] Coculture with tetraploid embryos

The rate of establishment of ES cells (including ES-like cells) from nuclear-transplanted embryos or male embryos is not particularly high. Even successfully established ES cell colonies do not keep favorable morphologies in many cases. To solve these problems, coculture with tetraploid embryos was performed.

First, the establishment of ES cells by the coculture of nuclear-transplanted embryos with tetraploid embryos will be described. The operation was performed by a usual electric fusion method. F1 female mice (8-week-old) obtained from mating between B6-mtMUS female mice and DBA male mice were subjected to superovulation treatment, and the obtained unfertilized eggs were enucleated and then fused with fibroblasts derived from the tails of B10mdx male mice. After activation with strontium, the eggs were cultured in M16 up to an 8-cell stage. Two blastomeres at the 8-cell stage were introduced into the tetraploid 4-cell-stage embryos (Example 4) before compaction by use of a micromanipulator. Sixteen chimeric embryos were constructed from four 8-cell-stage nuclear-transplanted embryos and cultured in M16. All the embryos grew to a blastocyst stage, in which inner cell mass (ICM) development was seen. Of them, 6 embryos were selected at random and used in the establishment of ES cells. Finally, 3 lines of ES cell lines could be established from the 6 embryos.

Male embryo-derived ES cells established aside from this formed oblate colonies and had a low proliferating ability. The male embryo production was performed by substituting the female pronuclei of pronuclear-stage embryos obtained from mating between F1 females after superovulation treatment and 129 male mice with the male pronuclei of pronuclear-stage embryos obtained from mating between F1 females after superovulation treatment and B6 male mice. Approximately 10 of these male embryo-derived ES cells were injected into the tetraploid blastocyst-stage embryos obtained in Example 4 by use of a piezo-micromanipulator. After culture for approximately 1 day, they were cultured by using the establishment method of Example 1. Although ICM masses usually proliferate around 5 culture days, ICM masses derived from ES cells proliferate around 3 culture days. ES cell colonies established by repeating this procedure several times had excellent morphologies and had a high proliferating ability.

### [Example 10] Coculture with tetraploid embryos and W^{v}/W^{v} ES cells

The fertilized embryos of KK mice were collected at the 8-cell stage from their uteruses. Two blastomeres at the 8-cell stage were introduced into the tetraploid 4-cell-stage embryos (Example 4) before compaction by use of a micromanipulator. Nineteen chimeric embryos were constructed from five 8-cell-stage nuclear-transplanted embryos and cultured in M16. All the embryos grew to a blastocyst stage, in which inner cell mass (ICM) development was seen. However, ES cells could not be established therefrom. Next, 12 chimeric embryos were constructed in the same way as above. Approximately 5 W^{v}/W^{v} ES cells treated with mitomycin were introduced into the chimeric embryos that became blastocyst-stage embryos after 1.5-day culture by use of a piezo-micromanipulator. When ES cells were established in the same way as in Example 1, 6 lines of ES cells could be established.

### [Example 11] Transplantation of androgenetic embryo-derived ES cells into seminiferous tubule

An experiment shown below was conducted for the purpose of transplanting androgenetic embryo-derived ES cells into a seminiferous tubule and examining their movements. Materials used were B6xBALB F2 nu/nu W^{v}/W^{v} and androgenetic embryo-derived ES cells (Line name: 1-1, which retained GFP and neo as markers; passage number 8 and reestablished). The procedures were as follows: first, female C57BL/6J W^{v}/+ and male BALB/cA nu/+ were mated to produce a W^{v}/+ and nu/+ F1 mouse (B6xBALB F1, W^{v}/+, nu/+). This mouse was mated to thereby produce a W^{v}/W^{v} and nu/nu F2 mouse (B6xBALB F2, W^{v}/W^{v}, nu/nu). Reestablished androgenetic embryo-derived ES cells were transplanted into the seminiferous tubule of this mouse that became 8 weeks old. The reestablishment was performed in the same way as in ES cell establishment by injecting approximately 10 androgenetic embryo-derived ES cells into blastodisc-stage embryos of tetraploid embryos.

Hereinafter, the details thereof will be described. First, cells are prepared. Androgenetic embryo-derived ES 1-1P8 in a confluent state after two-day culture on MEF treated in advance with mitomycin was used. The cells were washed with PBS (-) and then taken off the dish for cell culture by use of a trypsin/EDTA (0.25%/0.04%) solution. The reaction was terminated by the addition of an equal amount of 10% FBS-supplemented DMEM medium. The cells were dissociated by pipetting and then filtrated with a grid nylon mesh of 40 µm square. This cell suspension was made into a pellet with a centrifuge. This pellet was resuspended in a sufficient amount of an ES medium, and the number of the cells was determined with a hemacytometer. This cell suspension was made into a pellet again with a centrifuge and suspended in a medium for injection (85 µl of ES medium + 5 µl of 20 mg/ml DNase + 10 µl of trypan blue) to prepare 100 µl of 4.3 × 10⁷ cells/ml cell suspension.

Next, transplantation will be described. A B6xBALB F2 W^{v}/W^{v} nu/nu mouse was put under anesthesia by injecting 0.3 ml of anesthetic solution (Nembutal:100% ethanol:PBS=1:1:11). Its lower abdominal region was sterilized with 70% ethanol, and an incision was made on the midline portion. The testis was extracted by pinching fat around the testis with tweezers. The vas deferens was exposed under a stereoscopic microscope. A rubber bulb was used to fill a glass tube having an elongated tip with the cell suspension. The glass tube was placed on a mouthpiece for transplantation. While the vas deferens was held, the glass tube was pierced thereto, and the needle was allowed to enter into the rete testis to inject the cell suspension into the seminiferous tubule by expiration. The cell suspension was confirmed to be sufficiently filled, and then, the glass tube was removed. The testis was returned to the abdominal cavity. Transplantation was conducted on the remaining testis in the same way as above. The peritoneum and the muscular layer were stitched with suture, and the skins were secured with a metallic clip.

This time, the cell suspension could be injected in large amounts into the left testis by this procedure, while a sufficient amount of the cell suspension could also be injected into the right testis.

The testis of the B6xBALB F2 W^{v}/W^{v} nu/nu mouse was a very small testis specific to the W^{v}/W^{v} mouse. The testis went back to the original position on the next day of transplantation and grew to a size almost equal to that of a normal mouse after 1 month. As shown in Figure 8, a sperm standing in the middle of formation was seen. Accordingly, the androgenetic embryo-derived ES cells can be said to have the property of a sperm stem cell.

### [Example 12] Analysis of methylation pattern of androgenetic embryo-derived ES cells

The androgenetic embryo-derived ES cells (Androgenetic ES: AgES) used in the experiment and 3 lines of normal B6-derived ES cells serving as a comparative control group were used to confirm the state of genomic imprinting. The state of imprinting can be examined by measuring the methylation states of CpG sites of DMRs (Differentially Methylated Regions) located upstream of imprinted genes. First, DNA was extracted from each sample and subjected to bisulfite treatment using the CpGenome DNA Modification kit (Intergen). Next, to conduct combined bisulfite restriction analysis (COBRA), primers specific to imprinted genes Pegl/Mest, Snrpn, and Igf2r methylated in a maternally specific manner were used to amplify the DNA of the sample by PCR. The primers used are as follows:
Peg1/Mest (Promoter & exon 1, Genbank acc. no. AF017994),
5'-GGTTGGGTTTGGATATTGTAAAGT-3' (SEQ ID NO: 3) and
5'-TTCCCTATAAATATCTTCCCATATTC-3' (SEQ ID NO: 4)
Snrpn (DMR1, Genbank acc. no. AF081460),
5'-TTTGGTAGTTGTTTTTTGGTAGGATAT-3' (SEQ ID NO: 5) and
5'-ACTAAAATCCACAAACCCAACTAAC-3' (SEQ ID NO: 6)
Igf2r (DMR2, Genbank acc. no. L06446),
5'-GAAGTTGTGATTTTGGTTATGTTAAG-3' (SEQ ID NO: 7) and
5'-ACAATTTACACCCTCAAAATACCTC-3' (SEQ ID NO: 8)

The Peg1/Mest and Igf2r amplification products were subjected to restriction enzyme treatment with TaqI at 65°C for 4 hours, while the Snrpn amplification products were subjected to restriction enzyme treatment with HhaI at 37°C for 4 hours. These samples were electrophoresed on 2.5% agarose to confirm DNA fragment lengths by ethidium bromide staining.

COBRA probably provides 90-bp and 47-bp fragments cut from a 137-bp Peg1/Mest amplification product, 135-bp and 113-bp fragments cut from a 248-bp Snrpn amplification product, and a 141-bp fragment and several fragments cut from 193-bp Igf2r amplification product, if DNA is methylated. The normal ES cells used as a comparative control group gave approximately 50% methylated DNAs and exhibited the same pattern as that of a normal fertilized egg or somatic cell. On the other hand, the androgenetic embryo-derived ES cell gave no methylated imprinted genes and exhibited the same methylation pattern as that of a sperm.

### Industrial Applicability

A C-kit mutant animal (W gene mutant animal)-derived pluripotent cell incapable of forming a germ cell of the present invention is a pluripotent cell that could not be established previously. A pluripotent cell such as an ES cell having a high proliferating ability can be established by culturing the pluripotent cell of the present invention with a reproductive organ-derived cell such as a blastocyst cell of an animal. Moreover, the proliferating ability of an established totipotent cell can be kept high by coculturing the established pluripotent cell with the pluripotent cell incapable of forming a germ cell of the present invention. As a result, it is possible to use repetitively subcultured pluripotent cells in chimeric animal production and so on. Furthermore, a chimeric animal comprising an additional pluripotent cell transmitted to the germ line can be produced efficiently by injecting the pluripotent cell incapable of forming a germ cell of the present invention together with the additional pluripotent cell such as an ES cell into an early embryo of an animal, and then causing ontogenesis. It is also possible to control the sex ratio of born chimeric animals.

In a method for producing a chimeric animal of the present invention, comprising injecting into a host embryo of an animal, two or more kinds of cells comprising a pluripotent cell having a mutation or deletion of a particular gene and lacking a function involving the gene and an additional pluripotent cell, a chimeric animal can be allowed to produce a protein of an animal of a different species or to produce a visceral organ-specific stem cell or visceral organ of an animal of a different species by properly selecting the two or more kinds of cells comprising the pluripotent cell having a mutation or deletion of a particular gene and lacking a function involving the gene and the additional pluripotent cell.

All publications, patents, and patent applications cited herein are incorporated herein by reference in their entirety.
## Claims

1. A method for producing a chimeric animal, comprising injecting into a host embryo of an animal, two or more kinds of cells comprising a pluripotent cell having a mutation or deletion of a particular gene and lacking a function involving the gene and an additional pluripotent cell other than the pluripotent cell.

2. A method for producing a chimeric animal, comprising coculturing two or more kinds of cells comprising a pluripotent cell having a mutation or deletion of a particular gene and lacking a function involving the gene and an additional pluripotent cell other than the pluripotent cell, and then simultaneously injecting the cells into a host embryo of an animal.

3. The method for producing a chimeric animal according to claim 2, wherein the coculture is performed in an early embryo of an animal.

4. The method for producing a chimeric animal according to any one of claims 1 to 3, wherein the chimeric animal is a chimeric animal where a gene functions which is derived from the additional cell other than the pluripotent cell having a mutation or deletion of a particular gene and lacking a function involving the gene.

5. The method for producing a chimeric animal according to any one of claims 1 to 3, wherein the chimeric animal is a chimeric animal where a gene functions which is derived from the additional cell other than the pluripotent cell having a mutation or deletion of a particular gene and lacking a function involving the gene and corresponds to the gene mutated or deleted.

6. The method for producing a chimeric animal according to any one of claims 1 to 5, wherein the additional pluripotent cell other than the pluripotent cell having a mutation or deletion of a particular gene and lacking a function involving the gene is a genetically modified cell.

7. The method for producing a chimeric animal according to claim 6, wherein the genetic modification is introduction of a foreign gene or knockout of an endogenous gene.

8. The method for producing a chimeric animal according to any one of claims 1 to 7, wherein the pluripotent cell having a mutation or deletion of a particular gene and lacking a function involving the gene and/or the additional pluripotent cell are pluripotent cells comprising adapted mitochondrial DNA introduced therein or substituted for the original mitochondrial DNA.

9. The method for producing a chimeric animal according to any one of claims 1 to 8, wherein the host embryo of the animal is a host embryo comprising adapted mitochondrial DNA introduced therein.

10. The method for producing a chimeric animal according to any one of claims 1 to 9, wherein the host embryo of the animal is a blastocyst.

11. The method for producing a chimeric animal according to claim 10, wherein the host embryo of the animal is a tetraploid.

12. The method for producing a chimeric animal according to any one of claims 1 to 11, wherein the pluripotent cell is a cell selected from the group consisting of an ES cell, an EG cell, and a GS cell.

13. The method for producing a chimeric animal according to any one of claims 1 to 12, wherein the additional cell other than the pluripotent cell having a mutation or deletion of a particular gene and lacking a function involving the gene is of the same species in terms of zootaxy as the pluripotent cell having a mutation or deletion of a particular gene and lacking a function involving the gene.

14. The method for producing a chimeric animal according to any one of claims 1 to 12, wherein the additional cell other than the pluripotent cell having a mutation or deletion of a particular gene and lacking a function involving the gene is of a different species in terms of zootaxy from the pluripotent cell having a mutation or deletion of a particular gene and lacking a function involving the gene.

15. The method for producing a chimeric animal according to any one of claims 1 to 14, wherein the pluripotent cell having a mutation or deletion of a particular gene and lacking a function involving the gene is derived from an animal selected from the group consisting of a mouse, a rat, a pig, cattle, a horse, a sheep, a goat, and a monkey.

16. The method for producing a chimeric animal according to any one of claims 1 to 15, wherein the additional cell other than the pluripotent cell having a mutation or deletion of a particular gene and lacking a function involving the gene is derived from an animal selected from the group consisting of a mouse, a rat, a pig, cattle, a horse, a sheep, a goat, a monkey, and a human.

17. The method for producing a chimeric animal according to any one of claims 1 to 16, wherein in the pluripotent cell having a mutation or deletion of a particular gene and lacking a function involving the gene, the gene mutated or deleted is one or more genes selected from the group consisting of
(a) a gene involved in germ cell formation,
(b) a gene involved in the immune system, and
(c) a gene involved in formation of a particular tissue or organ or a gene encoding a particular protein.

18. The method for producing a chimeric animal according to any one of claims 1 to 16, wherein the pluripotent cell having a mutation or deletion of a particular gene and lacking a function involving the gene is a pluripotent cell incapable of forming a germ cell, which is obtained from interspecific crossing.

19. The method for producing a chimeric animal according to claim 17, wherein the pluripotent cell having a mutation or deletion of a particular gene and lacking a function involving the gene is a pluripotent cell having a mutation or deletion of a gene involved in germ cell formation and being incapable of forming a germ cell, and the additional pluripotent cell other than the pluripotent cell having a mutation or deletion of a particular gene and lacking a function involving the gene is transmitted to the germ line.

20. The method for producing a chimeric animal according to claim 19, wherein the pluripotent cell having a mutation or deletion of a gene involved in germ cell formation and being incapable of forming a germ cell is a C-kit mutant animal-derived pluripotent cell incapable of forming a germ cell.

21. The method for producing a chimeric animal according to claim 20, wherein the C-kit mutant animal is a W^{v}/W^{v} mouse.

22. The method for producing a chimeric animal according to any one of claims 19 to 21, wherein the additional pluripotent cell other than the pluripotent cell having a mutation or deletion of a particular gene and lacking a function involving the gene is a genetically modified cell, and the genetically modified pluripotent cell is transmitted to the germ line.

23. The method for producing a chimeric animal according to claim 22, wherein the genetic modification is introduction of a foreign gene or knockout of an endogenous gene.

24. The method for producing a chimeric animal according to claim 17, wherein the pluripotent cell having a mutation or deletion of a particular gene and lacking a function involving the gene is a pluripotent cell having a mutation or deletion of a gene involved in the immune system and having an impairment of immune function.

25. The method for producing a chimeric animal according to claim 24, wherein the pluripotent cell having a mutation or deletion of a gene involved in the immune system and having an impairment of immune function is a pluripotent cell having a mutation or deletion of a gene encoding an antibody and lacking an ability to produce an antibody.

26. The method for producing a chimeric animal according to claim 24, wherein the pluripotent cell having a mutation or deletion of a gene involved in the immune system and having an impairment of immune function is an immunodeficient animal-derived pluripotent cell.

27. The method for producing a chimeric animal according to claim 26, wherein the immunodeficient animal is a nu/nu mouse or scid mouse.

28. The method for producing a chimeric animal according to claim 24, wherein the pluripotent cell having a mutation or deletion of a gene involved in the immune system and having an impairment of immune function is a lymphocyte-deficient animal-derived pluripotent cell.

29. The method for producing a chimeric animal according to claim 28, wherein the lymphocyte-deficient animal is an animal having a mutation or deletion of a RAG-1 gene and/or a RAG-2 gene.

30. The method for producing a chimeric animal according to claim 17, wherein the pluripotent cell having a mutation or deletion of a particular gene and lacking a function involving the gene is a pluripotent cell having a mutation or deletion of a gene involved in formation of a particular tissue or organ or a gene encoding a particular protein.

31. The method for producing a chimeric animal according to claim 30, wherein a gene which is carried by the additional pluripotent cell other than the pluripotent cell having a mutation or deletion of a particular gene and lacking a function involving the gene and corresponds to the gene mutated or deleted causes a particular tissue or organ or particular protein involving the gene to be produced in the produced chimeric animal.

32. The method for producing a chimeric animal according to claim 17, wherein the pluripotent cell having a mutation or deletion of a particular gene and lacking a function involving the gene is a pluripotent cell having a mutation or deletion of a gene involved in the immune system and further having a mutation or deletion of a gene involved in germ cell formation or a gene involved in formation of a particular tissue or organ or a gene encoding a particular protein.

33. The method for producing a chimeric animal according to any one of claims 1 to 32, wherein the additional pluripotent cell other than the pluripotent cell having a mutation or deletion of a particular gene and lacking a function involving the gene is
(a) a fusion cell between the additional pluripotent cell other than the pluripotent cell having a mutation or deletion of a particular gene and lacking a function involving the gene and a cell of the same or a different species in terms of zootaxy as (from) the pluripotent cell having a mutation or deletion of a particular gene, or
(b) a pluripotent cell derived from a cell of the same or a different species in terms of zootaxy as (from) the pluripotent cell having a mutation or deletion of a particular gene.

34. The method for producing a chimeric animal according to claim 33, wherein the cell of the same or a different species in terms of zootaxy as (from) the additional pluripotent cell other than the pluripotent cell having a mutation or deletion of a particular gene and lacking a function involving the gene is a tissue stem cell or differentiated cell.

35. The method for producing a chimeric animal according to claim 33 or 34, wherein the method comprises injecting into the host embryo of the animal, a pluripotent cell having a mutation or deletion of a gene involved in germ cell formation and being incapable of forming a germ cell, and
(a) a fusion cell between the pluripotent cell having a mutation or deletion of a gene involved in germ cell formation and being incapable of forming a germ cell and a cell of the same or a different species in terms of zootaxy as (from) the pluripotent cell having a mutation or deletion of a gene involved in germ cell formation, or
(b) a pluripotent cell-like cell derived from a cell of the same or a different species in terms of zootaxy as (from) the pluripotent cell having a mutation or deletion of a gene involved in germ cell formation,
wherein the cell of the same or a different species is capable of being transmitted to the germ line.

36. The method for producing a chimeric animal according to claim 33 or 34, wherein the method comprises injecting into the host embryo of the animal, a pluripotent cell having a mutation or deletion of a gene involved in the immune system and having an impairment of immune function, and
(a) a fusion cell between the pluripotent cell having a mutation or deletion of a gene involved in the immune system and having an impairment of immune function and a cell of a different species in terms of zootaxy from the pluripotent cell, or
(b) a pluripotent cell-like cell derived from a cell of a different species from the pluripotent cell,
wherein the pluripotent cell having a mutation or deletion of a gene involved in the immune system and having an impairment of immune function and the cell of a different species contribute to formation of the chimeric individual.

37. A chimeric animal produced by a method according to any one of claims 1 to 36.

38. A method for producing a germ cell derived from an additional pluripotent cell other than a pluripotent cell having a mutation or deletion of a particular gene and lacking a function involving the gene, comprising collecting from a chimeric animal produced by a method according to any one of claims 17 to 23, a germ cell derived from an additional pluripotent cell other than a pluripotent cell having a mutation or deletion of a particular gene and lacking a function involving the gene.

39. A method for producing a genetically modified animal, comprising mating a chimeric animal produced by a method according to any one of claims 17 to 23 and obtaining a genetically modified animal as progeny.

40. A genetically modified animal produced by a method according to claim 39.

41. A method for producing an antibody, comprising administering an antigen to a chimeric animal or progeny thereof produced by a method according to any one of claims 17 and 24 to 29, wherein the chimeric animal or progeny thereof has a gene encoding an antibody derived from an additional pluripotent cell other than a pluripotent cell having a mutation or deletion of a gene involved in the immune system and having an impairment of immune function.

42. A method for producing a particular tissue or organ or a particular protein, comprising allowing a chimeric animal or progeny thereof produced by a production method according to any one of claims 17, 30, and 31 to form a particular tissue or organ or to produce a particular protein.

43. A pluripotent cell derived from a C-kit mutant animalwhich is incapable of forming a germ cell.

44. The pluripotent cell according to claim 43, wherein the C-kit mutant animal is a W^{v}/W^{v} mouse.

45. A pluripotent cell derived from an immunodeficient animalwhich has a mutation or deletion of a gene involved in immunity.

46. The pluripotent cell according to claim 45, wherein the animal is a nu/nu mouse or scid mouse.

47. A pluripotent cell derived from a lymphocyte-deficient animalwhich has a mutation or deletion of a gene involved in immunity.

48. The pluripotent cell according to claim 47, wherein the lymphocyte-deficient animal is an animal having a mutation or deletion of a RAG-1 gene and/or a RAG-2 gene.

49. The pluripotent cell according to any one of claims 43 to 48, wherein the pluripotent cell is a cell selected from the group consisting of an ES cell, an EG cell, and a GS cell.

50. The pluripotent cell according to any one of claims 43 to 49, wherein the pluripotent cell comprises adapted mitochondrial DNA introduced therein as mitochondrial DNA.

51. The pluripotent cell according to claim 50, wherein the adapted mitochondrial DNA is a wild-type mouse Mus musculus musculus type.

52. The pluripotent cell according to any one of claims 43 to 51, wherein the sex chromosomal type is a male XY type.

53. The pluripotent cell according to any one of claims 43 to 51, wherein the sex chromosomal type is a female XX type.

54. A method for establishing a pluripotent cell from a reproductive organ-derived cell by mixed-culturing a pluripotent cell according to any one of claims 43 to 53 with a cell derived from an early embryo or germ cell of an animal.

55. The method according to claim 54, wherein the early embryo of the animal is an androgenetic embryo or gynogenetic embryo.

56. The method for establishing a pluripotent cell from a cell derived from an early embryo or germ cell according to claim 54 or 55, wherein the mixed-culture is coculture in an early embryo of an animal.

57. The method for establishing a pluripotent cell according to any one of claims 54 to 56, wherein the cell derived from an early embryo or germ cell is a cell derived from an inner cell mass of a blastocyst, and the pluripotent cell is an ES cell.

58. The method for establishing a pluripotent cell according to any one of claims 54 to 56, wherein the cell derived from an early embryo or germ cell is a primordial germ cell, and the pluripotent cell is an EG cell.

59. The method for establishing a pluripotent cell according to any one of claims 54 to 56, wherein the cell derived from an early embryo or germ cell is a germ cell, and the pluripotent cell is a GS cell.

60. A method for improving a proliferating ability of an additional pluripotent cell, comprising coculturing a pluripotent cell according to any one of claims 43 to 53 with the additional pluripotent cell.

61. The method for improving a proliferating ability of an additional pluripotent cell according to claim 60, wherein the coculture is performed in an early embryo of an animal.

62. The method for improving a proliferating ability of a pluripotent cell according to claim 60 or 61, wherein the additional pluripotent cell is a cell selected from the group consisting of an ES cell, an EG cell, and a GS cell.

63. The method for improving a proliferating ability of a pluripotent cell according to any one of claims 60 to 62, wherein the additional pluripotent cell is a genetically modified pluripotent cell.

64. A method for producing a germ line chimeric animal comprising a germ cell derived from an additional pluripotent cell, comprising injecting a pluripotent cell according to any one of claims 43 to 53 together with the additional pluripotent cell into a host embryo of an animal.

65. The method for producing a germ line chimeric animal according to claim 64, wherein the pluripotent cell according to any one of claims 43 to 53 and/or the additional pluripotent cell are pluripotent cells comprising adapted mitochondrial DNA introduced therein or substituted for the original mitochondrial DNA.

66. The method for producing a germ line chimeric animal according to claim 64 or 65, wherein the host embryo of the animal is a host embryo comprising adapted mitochondrial DNA introduced therein or substituted for the original mitochondrial DNA.

67. The method for producing a germ line chimeric animal according to any one of claims 64 to 66, wherein the host embryo of the animal is a blastocyst.

68. The method for producing a germ line chimeric animal according to claim 67, wherein the host embryo of the animal is a tetraploid.

69. The method for producing a germ line chimeric animal according to any one of claims 64 to 68, wherein the additional pluripotent cell is a cell selected from the group consisting of an ES cell, an EG cell, and a GS cell.

70. The method for producing a germ line chimeric animal according to any one of claims 64 to 69, wherein the animal is a mouse.

71. The method for producing a germ line chimeric animal according to any one of claims 64 to 70, wherein the additional pluripotent cell is a genetically modified pluripotent cell.

72. A method for producing a germ line chimeric animal comprising a germ cell derived from an additional pluripotent cell, comprising coculturing a pluripotent cell according to any one of claims 43 to 53 with the additional pluripotent cell and injecting the cocultured cells together into a host embryo of an animal.

73. The method for producing a germ line chimeric animal according to claim 72, wherein the coculture is performed in an early embryo of an animal.

74. The method for producing a germ line chimeric animal according to claim 72 or 73, wherein the pluripotent cell according to any one of claims 43 to 53 and the additional pluripotent cell are pluripotent cells comprising adapted mitochondrial DNA introduced therein.

75. The method for producing a germ line chimeric animal according to any one of claims 72 to 74, wherein the host embryo of the animal is a host embryo comprising adapted mitochondrial DNA introduced therein.

76. The method for producing a germ line chimeric animal according to any one of claims 72 to 75, wherein the host embryo of the animal is a blastocyst.

77. The method for producing a germ line chimeric animal according to claim 76, wherein the host embryo of the animal is a tetraploid.

78. The method for producing a germ line chimeric animal according to any one of claims 72 to 77, wherein the additional pluripotent cell is a cell selected from the group consisting of an ES cell, an EG cell, and a GS cell.

79. The method for producing a germ line chimeric animal according to any one of claims 72 to 78, wherein the animal is a mouse.

80. The method for producing a germ line chimeric animal according to any one of claims 72 to 79, wherein the additional pluripotent cell is a genetically modified pluripotent cell.

81. A chimeric animal produced by a method according to any one of claims 64 to 80.

82. A method for producing a germ cell by transplanting an androgenetic embryo- or gynogenetic embryo-derived pluripotent cell established by a method according to claim 55 into the reproductive organ of an animal.

83. The method according to claim 82, wherein a sperm is produced by transplanting the androgenetic embryo-derived ES cell to the vas deferens of the animal.


Free Text of Sequence Listing SEQ ID NOs: 1 to 8: Primers

SEQUENCE LISTING

<110> Nagao, Yasumitsu
<110> Horii, Takuro

<120> A method for producing a chimera using totipotent cells

<130> PH-2538-PCT

<140>
<141>

<150> JP2004-211887
<151> 2004-07-20

<160> 8

<170> PatentIn Ver. 2.1

<210> 1
<211> 21
<212> DNA
<213> Artificial Sequence

<220>
<223> Description of Artificial Sequence: Primer

<400> 1
aaagagaggc cctaatgtcg g                                                21

<210> 2
<211> 19
<212> DNA
<213> Artificial Sequence

<220>
<223> Description of Artificial Sequence: Primer

<400> 2
ctcgagacta cctcccacc                                                    19

<210> 3
<211> 24
<212> DNA
<213> Artificial Sequence

<220>
<223> Description of Artificial Sequence: Primer

<400> 3
ggttgggttt ggatattgta aagt                                              24

<210> 4
<211> 26
<212> DNA
<213> Artificial Sequence

<220>
<223> Description of Artificial Sequence: Primer

<400> 4
ttccctataa atatcttccc atattc                                            26

<210> 5
<211> 27
<212> DNA
<213> Artificial Sequence

<220>
<223> Description of Artificial Sequence: Primer

<400> 5
tttggtagtt gttttttggt aggatat 27

<210> 6
<211> 25
<212> DNA
<213> Artificial Sequence

<220>
<223> Description of Artificial Sequence: Primer

<400> 6
actaaaatcc acaaacccaa ctaac 25

<210> 7
<211> 26
<212> DNA
<213> Artificial Sequence

<220>
<223> Description of Artificial Sequence: Primer

<400> 7
gaagttgtga ttttggttat gttaag 26

<210> 8
<211> 25
<212> DNA
<213> Artificial Sequence

<220>
<223> Description of Artificial Sequence: Primer

<400> 8
acaatttaca ccctcaaaat acctc                                          25